# EUROPEAN PATENT SPECIFICATION

(11) **EP 1 322 782 B1**
(45) Date of publication and mention of the grant of the patent: **26.11.2008**
(21) Application number: 01963280.1
(22) Date of filing: 10.09.2001
(51) Int. Cl.: C12Q 1/68

(54) **METHOD OF NUCLEIC ACID TYPING OR SEQUENCING**
METHODE ZUR TYPISIERUNG ODER SEQUENZIERUNG EINER NUKLEINSÄURE
METHODE DE TYPAGE OU SEQUENCAGE D'UN ACIDE NUCLEIQUE

(30) Priority: 08.09.2000 GB 0022069
(43) Date of publication of application: 02.07.2003
(73) Proprietor: Biotage AB, 753 18 Uppsala (SE); THE BOARD OF TRUSTEES OF THE LELAND STANFORD JUNIOR UNIVERSITY, Palo Alto, CA 93404-1850 (US)
(72) Inventor: RONAGHI, Mostafa, Stanford DNA Sequ & Technol Cent, Palo Alto, CA 94304 (US); EKSTRÖM, Björn, c/o PyroSequencing AB, S-752 28 Uppsala (SE); POURMAND, Nader, Stanford DNA Sequencing & Tech Ce, Palo Alto, CA 94304 (US)
(74) Representative: Dzieglewska, Hanna Eva
(86) International application number: PCT/GB2001/004042
(87) International publication number: WO 2002/020837

(56) References cited:
- WO-A-00/43540
- WO-A-00/50642
- ALDERBORN ANDERS ET AL: "Determination of single-nucleotide polymorphisms by real-time pyrophosphate DNA sequencing." GENOME RESEARCH, vol. 10, no. 8, August 2000 (2000-08), pages 1249-1258, XP002218192 ISSN: 1088-9051
- AHMADIAN AFSHIN ET AL: "Single-nucleotide polymorphism analysis by pyrosequencing." ANALYTICAL BIOCHEMISTRY, vol. 280, no. 1, 10 April 2000 (2000-04-10), pages 103-110, XP002193363 ISSN: 0003-2697
- SHI MICHAEL M: "Enabling large-scale pharmacogenetic studies by high-throughput mutation detection and genotyping technologies." CLINICAL CHEMISTRY, vol. 47, no. 2, February 2000 (2000-02), pages 164-172, XP002197957 ISSN: 0009-9147
- RONAGHI MOSTAFA ET AL: "A sequencing method based on real-time pyrophosphate" SCIENCE, vol. 281, no. 5375, pages 363-365, (WASHINGTON D C)

## Description

This invention relates to a method of typing using nucleic acid, and in particular to an improved method of genotyping.

Typing, e.g. genotyping, can be particularly advantageous for medical diagnosis, prognosis and treatment. For example, identification of the microbe responsible for infection allows correct treatment to be administered. It has been shown that microbes may now readily be identified by typing (i.e. by identifying genomic signature patterns characteristic of a particular microbe). Typing one or more variable regions in a gene or genes or other nucleic acid sequence of an individual can reveal markers of predisposition to a particular disease, condition or syndrome, and may also point to the best method of treatment of the foregoing. Typing methods are also useful for genomic analyses (e.g. in typing polymorphisms or allelic variations), tissue typing or environmental monitoring and contamination testing etc.

Large scale genotyping is discussed in Shi (Clinical Chemistry, 47:2, 164-172, 2001). This provides a review of recent technology such as Pyrosequencing^{™} in mutation detection and genotyping with a focus on genotyping of single nucleotide polymorphisms (SNP).

Conventional assays for detection of bacterial or viral species, or for detecting mutations or polymorphisms in a DNA sequence include using the polymerase chain reaction (PCR) method. This method is designed to permit selective amplification of a particular target DNA sequence or sequences, determined by the nature of the amplification primers used. To permit such selective amplification, some enabling the construction of two oligonucleotide primer sequences, known as amplimers. One amplimer hybridises at or towards the 5' end of one of the strands of the target DNA and the other amplimer at or towards the 5' end of the second strand. In the presence of a DNA polymerase and DNA precursors (i.e. dATP, dCTP, dGTP and dTTP) the primers can initiate the synthesis of new DNA strands which are complementary to the individual strands of the target DNA segment. The use of a heat-stable polymerase enables the procedure to be readily repeated or cycled. The newly synthesized DNA strands act as templates for further DNA synthesis in subsequent cycles. The reaction mixture is subjected to a temperature of about 90°C to separate the double stranded DNA formed by the polymerase. The reaction temperature is reduced to about 50°C to 70° to allow the single stranded DNA to anneal to the primers, and another round of DNA synthesis is performed. The DNA synthesized extends between the termini of the two primers. Preferably, the DNA polymerase used is thermophilic i.e. Taq polymerase. After 30 cycles of DNA synthesis, the products of PCR will include about 10⁵ copies of the specific target sequence. A typical PCR reaction cycle is therefore: synthesis of the separate strands by primer extension, separation of strands, primer annealing, synthesis of new strands.

The chain reaction can therefore be perpetuated merely by raising and lowering the temperature.

Typing (e.g. genotyping) be performed using PCR-based techniques, for example using allele-specific primers (Okamoto et al. 1992, Journal of General Virology, 73, 673-679; Widell, A et al. 1994, Journal of Medical Virology, 44, 272-279).

Currently, multiplex PCR may be used to screen samples of nucleic acids for a given panel of mutations/variations within the nucleic acid sequence. This method is still cumbersome for routine diagnostics, as the use of gel electrophoresis is essential. Alternative methods rely on the use of labelled nucleotides or primers, and may require complex detection strategies or mechanisms. There is thus a need for a typing method which may analyse nucleic acids with respect to 2 or more variable regions or positions typically without the need for gel electrophoresis and preferably without the use of labelled nucleotides or primers.

Other methods for typing include serologically-based detection methods (Viazov, et al. 1994, Journal of Virological Methods, 48, 81-91; Schroter, M., 1999, Journal of Medical Virology, 57, 230-234), line probe assay (Stuyver, L., 1993, Journal of General Virology, 74, 1093-1102, Stuyver, L., 1996, Transfusion, 36, 552-558), and restriction fragment length polymorphism (McOmish et al. 1993, Transfusion, 33, 7-13; Buoro, S. 1999, Intervirology, 42, 1-8) are well known in the art. However, sequencing continues to be regarded in the art as the "gold standard" method for typing. Accordingly, a sequencing-based typing method which avoids the drawbacks mentioned above would represent a considerable advance in the art.

Typing of different SNPs in parallel using Pyrosequencing^{™} is discussed in Aldeborn et al. (Genome Research, 10: 1249-1259, 2000). A sequencing primer is annealed to the target nucleic acid and separate reactions for different targets are performed in parallel using an automated sequencing instrument. Ahmadian et al. (Analytical Biochemistry 280, 103-110, 2000) discusses high throughput methods for analysis of SNP positions. Single SNPs are typed individually using the Pyrosequencing^{™} technique.

PCR is also commonly used in the detection of microbes, i.e. bacteria or viruses. However, conventional PCR assays are limited in diagnostic applications, and generally only indicate whether or not a microbe, or a particular sequence is present. For many infections, e.g. viral infections such as hepatitis C viral infection, the infecting microorganism may occur in a number of different sub-types, for example at least seven subtypes (or genotypes) are known of the HCV virus. It would be advantageous in such circumstances not only to determine that the general "class" (or genus or species) of infecting microorganism is present (e.g. HCV virus), but also to determine which of the sub-types is present.

Similarly, genomic studies have now revealed that many other diseases or disorders may be associated with genetic variations (e.g. mutations, allelic variations or polymorphisms (e.g. single nucleotide polymorphisms, (SNPs)), and that the presence of such variations may indicate a risk or predisposition to a disease or disorder, or may even indicate or predict how an individual may respond to a particular treatment for that disease or disorder (this latter effect is referred to as "pharmacogenomics"). Accordingly, in clinical science, the analysis (typing) of such variations may be of importance.

Microbial subtypes and clinically informative polymorphisms (or other genetic variations) frequently are characterised by combinations of genetic variations (i.e. variations in multiple (i.e. two or more) positions or regions of the genome etc.). Accordingly, for typing purposes in such situations it is necessary to "type" (or identify) more than one variation (polymorphism). In other words, it is necessary to type (or study or identify) a polymorphic pattern (a pattern of genetic variations) which pattern may cover more than one region of the genome to be studied. (The term "polymorphic pattern" is used herein broadly to include patterns, or combinations, of two or more (e.g. 3, 4, 5, 6, 7, 8, 9, 10 or more) of any type of genetic variation, e.g. mutations, allelic variants, polymorphisms of any type etc.). It will be understood that the variation can be an insertion or deletion of one or more nucleic acid residues.

Thus, for many microorganisms, diseases or predisposition to disease, an identification of the exact type of microbe, or genetic variation, present is needed to make a proper diagnosis or prognosis and in order to achieve this it is necessary to study more than one genetically variant position or region. As mentioned above, PCR is an extremely useful tool for the amplification and/or identification of a specific sequence of DNA, but to use conventional PCR techniques to determine the genotype of a nucleic acid molecule based on multiple genetic variations requires a repeated and multiple number of individual reactions to be performed, which would be cumbersome, time-consuming and expensive to perform using conventional technologies and procedures such as e.g. electrophoresis or labelling technologies. There is therefore a need for a typing assay that is accurate and reliable, has a short analysis time and is quick and easy to perform. The present invention addresses this need.

In particular, it has now been found that a simple, reliable, and accurate method for obtaining typing (sequence) information about a plurality of variable sites within target nucleic acid, may be performed using a primer extension reaction system using two or more specific primers designed to bind at or near to these variable sites, allowing primer extension reactions to be carried out on each primer annealed to a template nucleic acid sequence, either sequentially or simultaneously, and detecting the pattern of nucleotide incorporation in said primer extension reactions. The pattern of nucleotide incorporation providing the typing information about said variable sites.

This new method of the invention thus combines a multiplexing approach (i.e. an approach relying on the simultaneous or parallel performance of multiple reactions) with a particular strategy for detecting the result of the multiple primer extensions, namely detecting the pattern of nucleotide incorporation.

The method is particularly suited to automation e.g. in systems where reaction and reagent dispensing steps take place in a microtitre plate format. The methods are particularly suitable for identifying microbial species and subtypes thereof, but may also find application in other typing procedures e.g. typing of polymorphisms, e.g. for tissue typing or in clinical applications.

As described further below the present invention is advantageously based on a method of "sequencing-by-synthesis" (see e.g. US-A-4, 863, 849 of Melamede). This is a term used in the art to define sequencing methods which rely on the detection of nucleotide incorporation during a primer-directed polymerase extension reaction. The four different nucleotides (i.e. A, G, T or C nucleotides) are added cyclically or sequentially (conveniently in a known order), and the event of incorporation can be detected in various ways, directly or indirectly, This detection reveals which nucleotide has been incorporated, and hence sequencing information; when the nucleotide (base) which forms a pair (according to the normal rules of base pairing, A-T and C-G) with the next base in the template target sequence is added, it will be incorporated into the growing complementary strand (i.e. the extended primer) by the polymerase, and this incorporation will trigger a detectable signal, the nature of which depending upon the detection strategy selected.

Accordingly, the present invention provides a method of typing 1 or more nucleic acid molecules, said method comprising:
simultaneously hybridizing two or more extension primers to said nucleic acid molecule(s), and performing primer extension reactions therefrom, each primer binding at a different predetermined site in said nucleic acid molecule(s), and determining the pattern of nucleotide incorporation by sequencing-by-synthesis to obtain a test pattern (or "fingerprint") for said nucleic acid molecule which is optionally compared with one or more reference patterns to type the said nucleic acid molecule(s).

Preferably, the primer extension reactions occur simultaneously, i.e. both or all primers are annealed and are capable of primer extension at the same time. It will, of course, be appreciated that each individual primer can only be extended if a nucleotide is added to the reaction mix which is complementary to the next nucleotide in the template. Thus for each nucleotide addition, not every primer (or even any primer) will actually be extended and the term 'simultaneous' must be interpreted with this in mind.

The method of the invention may be used to type a nucleic acid molecule containing two or more sites at which its sequence may be variable ("variable sites") and each said primer binds at a site lying at or near to a variable site. Different nucleotides may be added sequentially to perform the primer extension reactions, and are described further below.

Alternatively, the method of the invention may be used to type two or more nucleic acid molecules containing 1 or more sites at which the sequence may be variable ("variable sites") and each primer binds at a site lying at or near to a variable site. Different nucleotides may be added sequentially to perform the primer extension reactions, and are described further below. This embodiment may be particularly useful when it is desired to obtain information about variable sites within the related genes, for example SNPs in Factor V Leiden and Prothrombin (FII) which are genetic risk factors for developing venous thrombosis.

The term "typing" as used herein includes any method of analysing the nucleotide sequence of the nucleic acid molecule to be analysed (i.e. the "test" or target nucleic acid). More particularly, the typing method of the invention includes methods for detecting, identifying or analysing genetic or sequence variation (e.g. genomic variation) in a target nucleic acid molecule or molecules (as mentioned above, this may be e.g. mutation, allelic variation, polymorphisms etc.). Methods of the invention thus include methods of identifying, differentiating or distinguishing a nucleic acid molecule or molecules. Since the typing method of the invention relies on detecting genetic variation in a nucleic acid molecule or molecules, it may be regarded as a method of genotyping. It will be understood that a nucleic acid molecule may itself be typed and also that a given variable site within a nucleic acid molecule may be typed.

"Genotyping" according to the present invention thus involves determining the genotype of the target nucleic acid molecule(s). In the context of this specification, the "genotype" may be regarded as the particular combination or pattern of the genetic variations which are studied or analysed in the method of the invention, which is exhibited (or expressed) by the nucleic acid molecule(s) in question. The genotype may thus comprise the combination (or pattern) of particular alleles (i.e. variations) which are found at the particular loci investigated.

In other words, the genotype is a combination or pattern of multiple genetic variations (or "variable sites") in target nucleic acid. The genetic variations which comprise or make up the genotype may be those selected for study in the method of the invention (notwithstanding that other genetic variations may also be present in the molecule, which are not investigated). As mentioned above, "multiple" as used herein means 2 or more (or 3,4,5,6,7,8,9,10 or more), and the genetic variations (or "variable sites") may be polymorphisms (e.g. SNPs), insertions, deletions, mutations, hypervariable regions, variable motifs, or allelic variations, etc. According to the methods of the invention, 2 or more, preferably 3 or more, e.g. 3-7 variable sites are investigated simultaneously. Unless two of such variable sites are found close together, e.g. with 50 nucleotides, preferably within 30 nucleotides, preferably within 20 nucleotides a separate primer will be required in order to type each variable site. So each primer will be responsible for generating typing information about one or more variable sites, a primer will therefore in effect have its 'own' variable site (s) .

Conveniently, the target nucleic acid may be DNA, although typing of RNA (e.g. mRNA) is also within the scope of the invention. If it is desired to type a RNA sample, the method may additionally include the step of generating cDNA from the RNA template, conveniently by using reverse transcriptase. Alternatively, if desired, the primer extension reactions may be performed directly on the RNA template.

The target nucleic acid may thus be any nucleic acid, isolated or synthetic, in any desired or convenient form. It may thus be genomic DNA, or isolated mRNA which may be used directly for analysis by the method of the invention, or it may be a nucleic acid product derived therefrom (or corresponding thereto), e.g. by synthesis, such as cDNA as mentioned above, or an amplification product (e.g. PCR amplicon), clones or library products etc.

The nucleic acid molecule(s) may be obtained or derived from any convenient source, which may be any material containing nucleic acid, and all biological and clinical samples are included as possible sources i.e. any cell or tissue samples of an organism, or any body fluid or preparation derived therefrom, as well as cell cultures, cell preparations, cell lysates etc. Environmental samples e.g. soil and water samples or food samples are also included. The samples may be freshly prepared or they may be prior-treated in any convenient way e.g. for storage.

Representative sources of nucleic acid thus include, for example, foods and allied products, clinical and environmental samples. However, the source will generally be a biological sample, which may contain any viral or cellular material, including all prokaryotic or eukaryotic cells, viruses, bacteriophages, mycoplasmas, protoplasts and organelles. Such biological material may thus comprise all types of mammalian and non-mammalian animal cells, plant cells, algae including blue-green algae, fungi, bacteria, protozoa etc. Representative sources thus include whole blood and blood-derived products such as plasma, serum and buffy coat, urine, faeces, cerebrospinal fluid or any other body fluids, tissues, cell cultures, cell suspensions etc.

The nucleic acid may be provided for investigation in any convenient form and conveniently will be contained in a sample, e.g. an aqueous sample (e.g. in a buffer etc.). The nucleic acid may be prepared for the typing method, as desired, according to techniques well known in the art, e.g. isolation, purification, cloning, copying, amplification, etc.

In carrying out the method of the invention, two or more primers ("extension primers") are provided which bind to the target nucleic acid at a predetermined site, each primer binding site being different, so that multiple different primer extension reactions are performed. The extension primers are designed or selected so that their extension products overlap (or comprise) a site (e.g. locus or region) of sequence variability (i.e. genetic variation) in the target nucleic acid. In other words, the primers bind to the target nucleic at, or near to (e.g. within 1 to 40, 1 to 20, 1 to 10, or 1 to 6 bases of), a variable site. As mentioned above, such variable sites constitute the genotype of the target nucleic acid.

At least two extension primers are required to carry out the method, preferably at least three. However, the number of primers may be varied according to choice, for example, depending on the complexity of the system under study, and the detail of the information it is desired to obtain. Thus, for example, 3, 4, 5, or 6, or more extension primers (e.g. 3 to 15, or 3-10) may be used.

Thus, the term "variable site" refers to a site (e.g. locus or region) of a nucleic acid molecule which can differ in different genotypes. As defined above, the variable site may be a polymorphism or motif etc. Nucleic acid markers used for typing normally contain both conserved/semi-conserved and variable regions. Thus, each "type" will comprise a region of sequence variation, wherein this region (i.e. the sequence, or base identity, at that site) can be different from other types. In the method of the invention, at least two potential variable sites are examined, and, when one target nucleic acid molecule is typed, said nucleic acid molecule thus contains 2 or more (i.e. multiple) variable sites. Where 2 or more target nucleic acid molecules are typed, said nucleic acid molecules thus each contain 1 or more variable sites.

It will be understood by the skilled person in the art that any desired combination of variable sites can be analysed by the method of the invention. The variable sites do not have to be restricted to a single gene, coding region, non-coding region or nucleic acid molecule, but may be found anywhere in the target genome. It will further be understood that the variable site can be of any length, optionally 1 to 20 nucleotides, preferably 1 to 10 nucleotides in length. Typically, however, the variable site may comprise only a single or a few (e.g. 1-6, e.g. 1, 2, 3, 4, 5 or 6) nucleotides at which the sequence of the target nucleic acid may be variable. Thus, for example, a virus such as hepatitis C virus (HCV) may contain regions which are conserved between sub-types, but which nonetheless contain sites which may vary between subtypes. Such variable sites (which may typically be 1 to 3 nucleotides in length) may thus be used to distinguish between the various subtypes. In HCV such a conserved region containing variable sites is the 5' untranslated region (5'UTR), and this may conveniently be used in a genotyping assay method of the invention, as described further in Example 1 below.

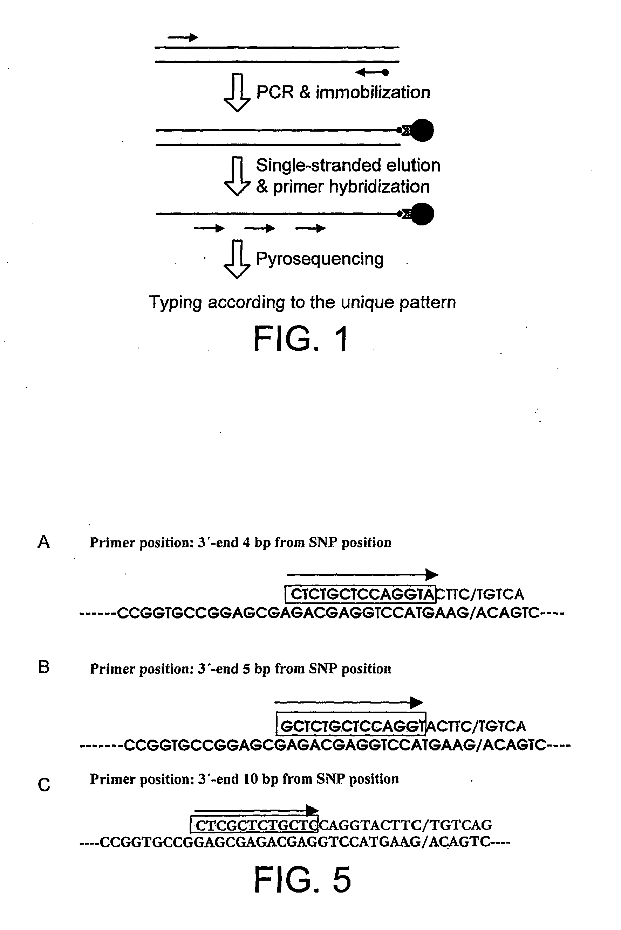

Other microorganisms will analogously have similar such regions in their genomes, containing variable sites, which may similarly be used in the method of the invention. For other typing applications e.g. typing of polymorphisms, regions of sequence variability, analogously containing polymorphic sites, may similarly be identified. For example, SNPs in the Renin-Angiotensinogen-Aldosterone system (RAAS) may be assessed using primers position in conserved regions of the genes. The primer can be position at or near to the SNP site. Figure 5 shows the positioning of three different extension primers, wherein the 3' end of the primer is 4 bases, 5 bases or 10 bases from the SNP position. The SNP is EU6 (ACE T3409C).

It will be understood that in order to perform the invention the primer binding sites should be available in all possible variants (genotypes) of the nucleic acid molecule(s) under study. Such primer binding sites will therefore advantageously lie in regions which are common to, or substantially conserved between, the different variants. This may readily be achieved by selecting the primer binding sites to lie in conserved/semi-conserved regions as discussed above.

The primer extension reactions conveniently may be performed by sequentially adding the nucleotides to the reaction mixture (i.e. a polymerase, and primer/template mixture). Advantageously the different nucleotides are added in known order, and preferably in a pre-determined order. In a convenient embodiment of the invention described in Example 1 below, the 4 different nucleotides (i.e. A, G, T and C nucleotides) are added sequentially in a predetermined order of addition. It thus forms a preferred aspect of the invention that the nucleotides are added sequentially in a predetermined order of addition. Therefore, the order of addition can be tailored to the nucleic acid(s) to be typed and the primers used. It will therefore be seen that the order of addition will not necessarily be cyclical e.g. A T G C A T G C but can be e.g. C G C T A G A.

As each nucleotide is added, it may be determined whether or not nucleotide incorporation takes place.

Advantageously, as described in more detail below, it may further be determined the amount (i.e. how many) of each nucleotide incorporated. In this manner, the pattern of nucleotide incorporation may be determined. In other words, the step of determining the pattern of nucleotide incorporation may comprise determining (or detecting) whether or not, and which, nucleotide is incorporated. Advantageously, this step also includes determining the amount of each nucleotide incorporated. Such a quantitative embodiment, wherein nucleotide incorporation is determined quantitatively, represents a preferred aspect of the invention.

In this manner, a "pattern" or "fingerprint" may be obtained for the target nucleic acid. This pattern comprises the base identity (i.e. sequence) of the particular variable sites identified for that nucleic acid molecule. In other words, the pattern corresponds to the genotype of the target nucleic acid. The genotype may thus readily be identified by comparing the pattern obtained to a reference pattern (or a "standard pattern"), or a panel of reference patterns (i.e. one or more, e.g. two or more e.g. 1 to 20, 1 to 15, 1 to 10, 1 to 6 or 1 to 3). A reference pattern may readily be obtained by determining the pattern of nucleotide incorporation using the extension primers in question on reference nucleic acid molecules of known genotype (e.g. a known microbial subtype or a known polymorphic pattern).

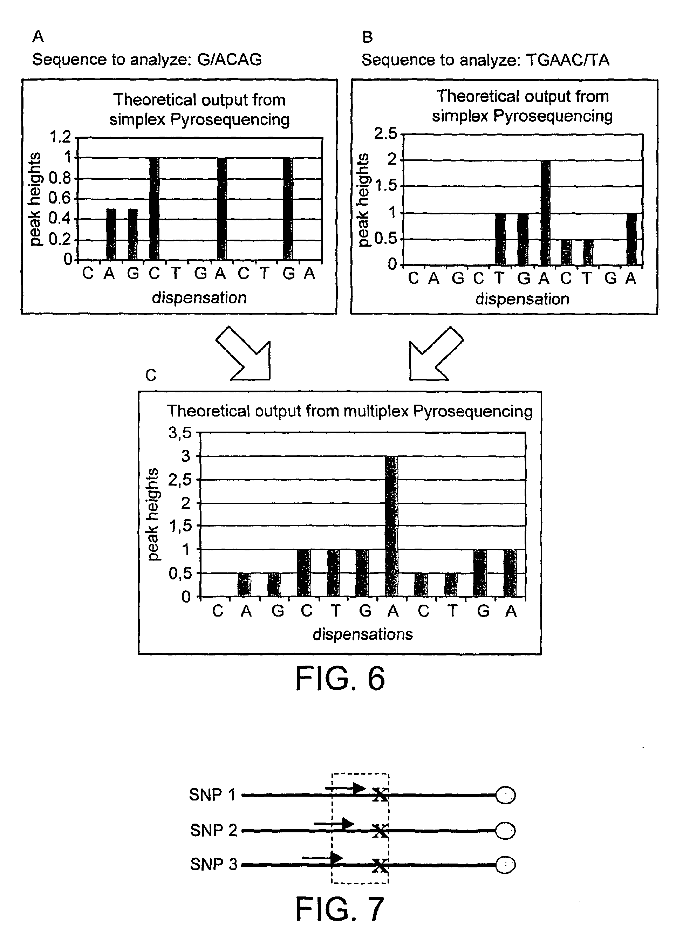

Alternatively, the 'reference pattern' can be theoretically derived from knowledge of the variable sites, as shown in the later Examples. It may then not be necessary actually to compare the pattern obtained with a reference pattern, the desired typing/sequence information can be read from the pattern obtained. Once the extension primers for each variable site have been selected and the order of addition of nucleotides determined, it is possible to determine a theoretical output from a primer extension reaction. Figure 6 shows the theoretical output from sequencing two variable sites individually, and the combination of extending both extension primers simultaneously. The theoretical reference pattern is shown for 2 variable sites present as heterozygotes. The primers used bound 3' to the sequences shown.

Thus, by identifying (or recognising) the pattern obtained for a target nucleic acid molecule, the genotype of the molecule may be identified (or recognised). Conveniently, test patterns and reference patterns may be compared using pattern recognition software.

In order to perform the invention, it may be advantageous or convenient first to amplify the nucleic acid molecule by any suitable amplification method known in the art. The target nucleic acid would then be an amplicon. Suitable in vitro amplification techniques include any process which amplifies the nucleic acid present in the reaction under the direction of appropriate primers. The amplicon method may thus preferably be PCR, or any of the various modifications thereof e.g. the use of nested primers, although it is not limited to this method. Those skilled in the art will appreciate that other amplification procedures may also be used, such as Self-sustained Sequence Replication (3SR), NASBA, the Q-beta replicase amplification system and Ligase chain reaction (LCR) (see for example Abramson and Myers (1993) Current Opinion in Biotech., 4: 41-47).

If PCR is used to amplify the nucleic acid, suitable primers, as discussed previously, are designed to ensure that the region of interest within the nucleic acid sequence (i.e. the region containing the variable sites), is amplified. PCR can also be used for indiscriminate amplification of all DNA sequences, allowing amplification of essentially all sequences within the sample for study (i.e. total DNA). Linker-primer PCR is particularly suitable for indiscriminate amplification, and uses double stranded oligonucleotide linkers with a suitable overhanging end, which are ligated to the ends of target DNA fragments. Amplification is then conducted using oligonucleotide primers which are specific for the linker sequences. Alternatively, completely random oligonucleotide primers may be used in conjunction with DOP-PCR (degenerate oligonucleotide-primed) to amplify all the DNA within a sample. If the variant sites to be typed by the method of the invention are present in discrete areas of the genome, multiplex PCR can be used to amplify nucleic acid sequences from the genome containing the variable sites. Therefore, multiple fragments can be amplified in a single PCR reaction.

In the method of the invention, several sequences may need to be amplified, to allow several regions (e.g. containing different variable sites) to be analysed. Therefore, several appropriate amplification primers may need to be synthesized to allow the selective amplification of several sequences in the target nucleic acid. It will therefore be understood that a number of different nucleic acid molecules may be present in the reaction mixture.

One or more of the amplification primers used in the amplification reaction, may be subsequently used as an "extension primer", but this will preferably be a different primer.

It will be appreciated that the sequence and length of the oligonucleotide amplification and extension primers to be used in the amplification and extension steps, respectively, will depend on the sequence of the target nucleic acid, the desired length of amplification or extension product, the further functions of the primer (i.e. for immobilization) and the method used for amplification and/or extension. Appropriate primers may readily be designed applying principles and techniques well known in the art.

Advantageously, as mentioned above, extension primers will bind near (e.g. within 1-40, 1-20, 1-10 or 1-6, preferably within 1-3 bases), substantially adjacent or exactly adjacent to the variable site of the target nucleic acid and will be complementary to a conserved or semi-conserved region of the nucleic acid. In certain embodiments, as described in Example 1 for instance, all primers will bind substantially adjacent to variable sites within the target nucleic acid (i.e. adjacent or within 3 bases of the variable site). In other embodiments, see for example Example 3, the primers will be staggered so that one is very close to its variable site, another is some distance away, e.g. 4-10 nucleotides distant and a third primer is 7 or more e.g. 8-16 nucleotides distant from its (first) variable site. Figure 7 depicts this principle.

In order for the method of the invention to be performed, knowledge of the sequence of the conserved or semi-conserved region is required in order to design an appropriate complementary extension primer. An extension primer is provided for each of the variable regions, each being specific for a site at or near to the variable site. The specificity is achieved by virtue of complementary base pairing. For all embodiments of the invention, primer design may be based upon principles well known in the art. It is not necessary for the extension or amplification primer to have absolute complementarily to the binding site, but this is preferred to improve the specificity of binding.

The extension primer may be designed to bind to the sense or anti-sense strand of the target nucleic acid.

In a preferred embodiment of the invention, the extension primers are designed to bind to the target nucleic acid near to the variable sites in such a way that upon the addition of nucleotides in a predetermined manner, the typing of each variable site takes place discretely. Thus analysis of a given variable site is not complicated by a positive incorporation signal from other variable or conserved regions. As shown in Example 1, it is possible to interpret the test pattern and allow for signals from nucleotide incorporation at more than the primer, but preferably when one primer is extending over a variable site, the other primers will be silent. Thus, if nucleotide incorporation takes place at one variable site, there is preferably no nucleotide incorporation at the other variable site(s). For example in the theoretical pattern shown in Figure 6, the extension primers are positioned in such a way that, upon the pre-determined sequential addition of nucleotides, each variable site is typed discretely, even though primer extension occurs simultaneously at other points - e.g. the second dispensation of nucleotide A. In this preferred embodiment, only the variable site is sequenced when an extended primer reaches its variable site; the other primers are not extended as the nucleotide added to the reaction mixture is not complementary to the next base in the templates for the other primer-extension reactions. Thus, the primers should be designed in parallel with the order of addition of the nucleotides. Primer design software can be used to determine the actual sequence of the primer once the 3' end has been fixed. Preferably, Pyrosequencing Primer Design Software is used.

Figure 7 shows a simplified set of multiplex primer extension reactions wherein the extension primers are placed at differing distances from the variant site (shown as X). This enables a pre-determined pattern of nucleotide addition to be performed wherein the variant site is sequenced in isolation. As can be envisaged, variations in the nucleotide sequence upstream of the different variable sites may mean that the primers need not anneal in such a staggered manner but the pattern of nucleotide addition alone may be sufficient to ensure the (extended) primers approach and/or sequence their variable sites at different times. Thus all primers may anneal at similar distances from the variable sites (or where one primer extension reaction is used to type 2 variable sites, the first variable site) e.g. substantially adjacent thereto, but where the nucleotides immediately upstream of and within the variable sites vary, so the order of nucleotide addition will control the order of primer extension across the variable sites.

The "primer extension" reaction according to the invention includes all forms of template-directed polymerase-catalysed nucleic acid synthesis reactions. Conditions and reagents for primer extension reactions are well known in the art, and any of the standard methods, reagents and enzymes etc. may be used in this step (see e.g. Sambrook et al., (eds), Molecular Cloning: a laboratory manual (1989), Cold Spring Harbor Laboratory Press). Thus, the primer extension reaction at its most basic, is carried out in the presence of primer, deoxynucleotides (dNTPs) and a suitable polymerase enzyme e.g. T7 polymerase, Klenow or Sequenase Ver 2.0 (USB USA), or indeed any suitable available polymerase enzyme. As mentioned above, for an RNA template, reverse transcriptase may be used. Conditions may be selected according to choice, having regard to procedures well known in the art.

The primer is thus subjected to a primer-extension reaction in the presence of a nucleotide, whereby the nucleotide is only incorporated if it is complementary to the base immediately adjacent (3') to the primer position. The nucleotide may be any nucleotide capable of incorporation by a polymerase enzyme into a nucleic acid chain or molecule. Thus, for example, the nucleotide may be a deoxynucleotide (dNTP, deoxynucleoside triphosphate) or dideoxynucleotide (ddNTP, dideoxynucleoside triphosphate). Thus, the following nucleotides may be used in the primer-extension reaction: guanine (G), cytosine (C), thymine (T) or adenine (A) deoxy- or dideoxy-nucleotides. Therefore, the nucleotide may be dGTP (deoxyguanosine triphosphate), dCTP (deoxycytidine triphosphate), dTTP (deoxythymidine triphosphate) or dATP (deoxyadenosine triphosphate). As discussed further below, suitable analogues of dATP, and also for dCTP, dGTP and dTTP may also be used. Modified nucleotides which include an activation or detectable group, radio or fluoroscently labelled nucleotide triphosphates can also be used in the primer extension step. Dideoxynucleotides may also be used in the primer-extension reaction. The term "dideoxynucleotide" as used herein includes all 2'-deoxynucleotides in which the 3' hydroxyl group is modified or absent. Dideoxynucleotides are capable of incorporation into the primer in the presence of the polymerase, but cannot enter into a subsequent polymerisation reaction, and thus function as a "chain terminator".

If the nucleotide is complementary to the target base, the primer is extended by one nucleotide, and inorganic pyrophosphate is released. As discussed further below, in a preferred method, the inorganic pyrophosphate may be detected in order to detect the incorporation of the added nucleotide. For some variable sites, the addition of one nucleotide will be sufficient to generate typing information. However, for the majority of variable sites, data for several adjacent nucleotides will be necessary. The extended primer can serve in exactly the same way in a repeated procedure to determine the next base in the variable region, thus permitting the whole variable site to be sequenced. Different nucleotides may be added sequentially, advantageously in known order, as discussed above, to reveal the nucleotides which are incorporated for each extension primer. Furthermore, in the case where the variable site is homopolymeric (i.e. contains 2 or more identical bases), the number of nucleotides incorporated of the complementary base will reflect the number present in the homopolymeric region. Accordingly, determining the number of nucleotides incorporated for each nucleotide addition, will reveal this information, and hence contribute to the pattern of nucleotide incorporation.

Hence, a primer extension protocol may involve annealing a primer as described above, adding a nucleotide, performing a polymerase-catalysed primer extension reaction, detecting the presence or absence of incorporation of said nucleotide (and advantageously also determining the amount of each nucleotide incorporated) and repeating the nucleotide addition and primer extension steps etc. one or more times. As discussed above, single (i.e. individual) nucleotides may be added successively to the same primer-template mixture, or to separate aliquots of primer-template mixture, etc. according to choice.

In order to permit the repeated or successive (iterative) addition of nucleotides in a primer-extension procedure, the previously-added nucleotide must be removed. This may be achieved by washing, or more conveniently, by using a nucleotide-degrading enzyme, for example as described in detail in WO98/28440.

Accordingly, in a principal embodiment of the present invention, a nucleotide degrading enzyme is used to degrade any unincorporated or excess nucleotide. Thus, if a nucleotide is added which is not incorporated (because it is not complementary to the target base), or any added nucleotide remains after an incorporation event (i.e. excess nucleotides) then such unincorporated nucleotides may readily be removed by using a nucleotide-degrading enzyme. This is described in detail in WO98/28440.

The term "nucleotide degrading enzyme" as used herein includes any enzyme capable of specifically or non-specifically degrading nucleotides, including at least nucleoside triphosphates (NTPs), but optionally also di- and mono-phosphates, and any mixture or combination of such enzymes, provided that a nucleoside triphosphatase or other NTP-degrading activity is present. Where a chain terminating nucleotide is used (e.g. a dideoxy nucleotide is used), the nucleotide degrading enzyme should also degrade such a nucleotide. Although nucleotide-degrading enzymes having a phosphatase activity may conveniently be used according to the invention, any enzyme having any nucleotide or nucleoside degrading activity may be used, e.g. enzymes which cleave nucleotides at positions other than at the phosphate group, for example at the base or sugar residues. Thus, a nucleoside triphosphate degrading enzyme is essential for the invention. Nucleoside di-and/or mono-phosphate degrading enzymes are optional and may be used in combination with a nucleoside triphosphate degrading enzyme.

The preferred nucleotide degrading enzyme is apyrase, which is both a nucleoside diphosphatase and triphosphatase, catalysing the reactions NTP → NDP + Pi and NDP → NMP + Pi (where NTP is a nucleoside triphosphate, NDP is a nucleoside diphosphate, NMP is a nucleotide monophosphate and Pi is inorganic phosphate). Apyrase may be obtained from the Sigma Chemical Company. Other possible nucleotide degrading enzymes include Pig Pancreas nucleoside triphosphate diphosphorydrolase (Le Bel et al., 1980, J. Biol. Chem., 255, 1227-1233). Further enzymes are described in the literature.

The nucleotide-degrading enzyme may conveniently be included during the polymerase (i.e. primer extension) reaction step. Thus, for example the polymerase reaction may conveniently be performed in the presence of a nucleotide-degrading enzyme. Although less preferred, such an enzyme may also be added after nucleotide incorporation (or non-incorporation) has taken place, i.e. after the polymerase reaction step.

Thus, the nucleotide-degrading enzyme (e.g. apyrase) may be added to the polymerase reaction mixture (i.e. target nucleic acid, primer and polymerase) in any convenient way, for example prior to or simultaneously with initiation of the reaction, or after the polymerase reaction has taken place, e.g. prior to adding nucleotides to the sample/primer/polymerase to initiate the reaction, or after the polymerase and nucleotide are added to the sample/primer mixture.

Conveniently, the nucleotide-degrading enzyme may simply be included in the reaction mixture for the polymerase reaction, which may be initiated by the addition of the nucleotide.

According to the present invention, detection of nucleotide incorporation can be performed in a number of ways, such as by incorporation of labelled nucleotides which may subsequently be detected, or by using labelled probes which are able to bind to the extended sequence.

The method is performed using a sequencing-by-synthesis method, due to the fact that the extension reactions are quantitative, i.e. that the nucleotide incorporation may be determined quantitatively. As mentioned above, sequencing-by-synthesis methods are disclosed extensively in US-A-4,863,849, which discloses a number of ways in which activated nucleotide incorporation may be determined or detected, e.g. spectrophotometrically or by fluorescent detection techniques, for example by determining the amount of nucleotide remaining in the added nucleotide feedstock, following the nucleotide incorporation step. In a sequencing by synthesis reaction, determination of the pattern of nucleotide incorporation occurs simultaneously with primer extension. One working definition of Sequencing by synthesis is a method in which a single activated (i.e. labelled) nucleotide is or is not incorporated into a primed template, incorporation being detected by any suitable means. This step is repeated by addition of a different activated nucleotide and incorporation is again detected. These steps are repeated and from the sum of incorporated nucleotides, the sequence can be deduced. The preferred method of sequencing by synthesis is however a pyrophosphate detection-based method.

Preferably, therfore, nucleotide incorporation is detected by detecting PPi release, preferably by luminometric detection, and especially by bioluminonetric detection

PPi can be determined by many different methods and a number of enzymatic methods have been described in the literature (Reeves et al., (1969), Anal. Biochem., 28, 282-287; Guillory et al., (1971), Anal. Biochem., 39, 170-180 Johnson et al., (1968), Anal. Biochem., 15, 273; Cook et al., (1978), Anal. Biochem. 91, 557-565; and Drake et al., (1979), Anal. Biochem, 94, 117-120.)

It is preferably to use luciferase and luciferin in combination to identify the release of pyrophosphate since the amount of light generated is substantially proportional to the amount of pyrophosphate released which, in turn, is directly proportional to the amount of nucleotide incorporated. The amount of light can readily be estimated by a suitable light sensitive device such as luminometer. Thus, luminometric methods offer the advantage of being able to be quantitative.

Luciferin-luciferase reactions to detect the release of PPi are well known in the art. In particular, a method for continuous monitoring of PPi release based on the enzymes ATP sulphurylase and luciferase has been developed (Nyrén and Lundin, Anal. Biochem., 151, 504-509, 1985; Nyrén P., Enzymatic method for continuous monitoring of DNA polymerase activity (1987) Anal. Biochem Vol 167 (235-238)) and termed ELIDA (Enzymatic Luminometric Inorganic Pyrophosphate Detection Assay). The use of the ELIDA method to detect PPi is preferred according to the present invention. The method may however be modified, for example by the use of a more thermostable luciferase (Kaliyama et al., 1994, Biosci. Biotech. Biochem., 58, 1170-1171) and/or ATP sulfurylase (Onda et al., 1996, Bioscience, Biotechnology and Biochemistry, 60:10, 1740-42). This method is based on the following reactions: 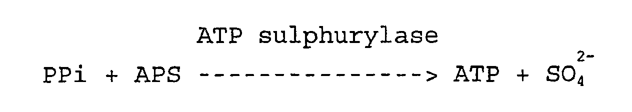 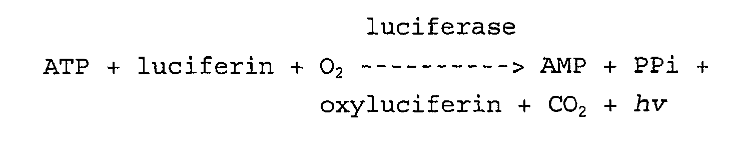 (APS = adenosine 5'-phosphosulphate)
Reference may also be made to WO 98/13523 and WO 98/28448, which are directed to pyrophosphate detection-based sequencing procedures, and disclose PPi detection methods which may be of use in the present invention.

In a PPi detection reaction based on the enzymes ATP sulphurylase and luciferase, the signal (corresponding to PPi released) is seen as light. The generation of the light can be observed as a curve known as a Pyrogram^{™}. Light is generated by luciferase action on the product, ATP (produced by a reaction between PPi and APS (see below) mediated by ATP sulphurylase) and, where a nucleotide-degrading enzyme such as apyrase is used, this light generation is then "turned off" by the action of the nucleotide-degrading enzyme, degrading the ATP which is the substrate for luciferase. The slope of the ascending curve may be seen as indicative of the activities of DNA polymerase (PPi release) and ATP sulphurylase (generating ATP from the PPi, thereby providing a substrate for luciferase). The height of the signal is dependent on the activity of luciferase, and the slope of the descending curve is, as explained above, indicative of the activity of the nucleotide-degrading enzyme. As explained below, Pyrogram^{™}in the context of a homopolymeric region, peak height is also indicative of the number of nucleotides incorporated for a given nucleotide addition step. Then, when a nucleotide is added, the amount of PPi released will depend upon how many nucleotides (i.e. the amount) are incorporated, and this will be reflected in the slope height.

Advantageously, by including the PPi detection enzyme(s) (i.e. the enzyme or enzymes necessary to achieve PPi detection according to the enzymatic detection system selected, which in the case of ELIDA, will be ATP sulphurylase and luciferase) in the polymerase reaction step, the method of the invention may readily be adapted to permit extension reactions to be continuously monitored in real-time, with a signal being generated and detected, as each nucleotide is incorporated.

Thus, the PPi detection enzymes (along with any enzyme substrates or other reagents necessary for the PPi detection reaction) may simply be included in the polymerase reaction mixture.

A potential problem which has previously been observed with PPi-based sequencing methods is that dATP, used in the chain extension reaction, interferes in the subsequent luciferase-based detection reaction by acting as a substrate for the luciferase enzyme. This may be reduced or avoided by using, in place of deoxyadenosine triphosphate (ATP), a dATP analogue which is capable of acting as a substrate for a polymerase but incapable of acting as a substrate for a PPi-detection enzyme. Such a modification is described in detail in WO98/13523.

The term "incapable of acting" includes also analogues which are poor substrates for the detection enzymes, or which are substantially incapable of acting as substrates, such that there is substantially no, negligible, or no significant interference in the PPi detection reaction.

Thus, a further preferred feature of the invention is the use of a dATP analogue which does not interfere in the enzymatic PPi detection reaction but which nonetheless may be normally incorporated into a growing DNA chain by a polymerase. By "normally incorporated" is meant that the nucleotide is incorporated with normal, proper base pairing. In the preferred embodiment of the invention where luciferase is a PPi detection enzyme, the preferred analogue for use according to the invention is the [1-thio]triphosphate (or α-thiotriphosphate) analogue of deoxy ATP, preferably deoxyadenosine [1-thio] triphospate, or deoxyadenosine α-thiotriphosphate (dATPαS) as it is also known. dATPαS, along with the α-thio analogues of dCTP, dGTP and dTTP, may be purchased from Amersham Pharmacia. Experiments have shown that substituting dATP with dATPαS allows efficient incorporation by the polymerase with a low background signal due to the absence of an interaction between dATPαS and luciferase. False signals are decreased by using a nucleotide analogue in place of dATP, because the background caused by the ability of dATP to function as a substrate for luciferase is eliminated. In particular, an efficient incorporation with the polymerase may be achieved while the background signal due to the generation of light by the luciferin-luciferase system resulting from dATP interference is substantially decreased. The dNTPαS analogues of the other nucleotides may also be used in place of the other dNTPs.

Another potential problem which has previously been observed with sequencing-by-synthesis methods is that false signals may be generated and homopolymeric stretches (i.e. CCC) are difficult to sequence with accuracy. This may be overcome by the addition of a single-stranded nucleic acid binding protein (SSB) once the extension primers have been annealed to the template nucleic acid. The use of SSB in sequencing-by-synthesis is discussed in WO 00/43540 of *Pyrosequencing* AB.

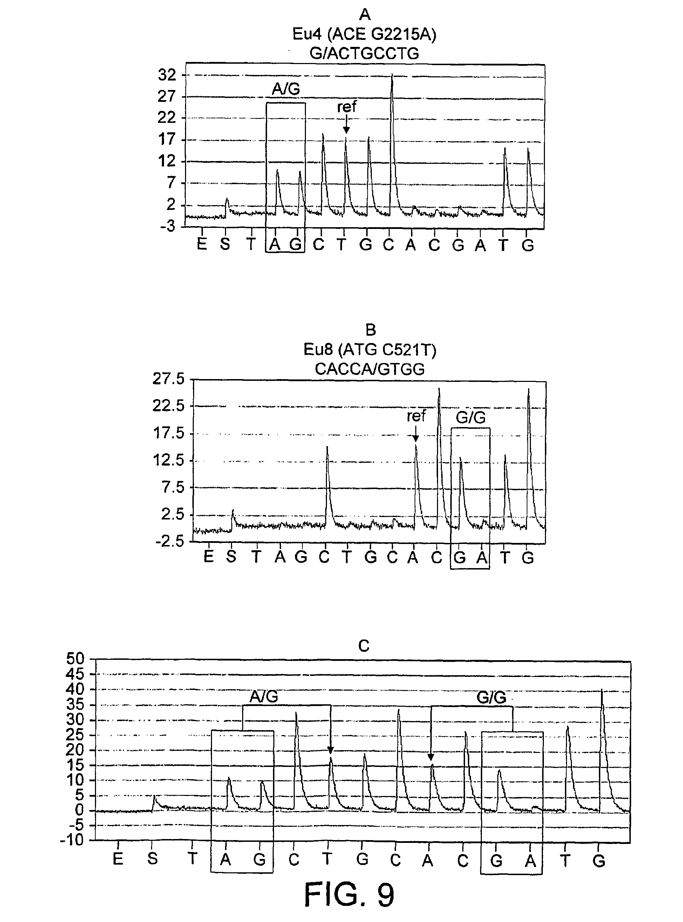

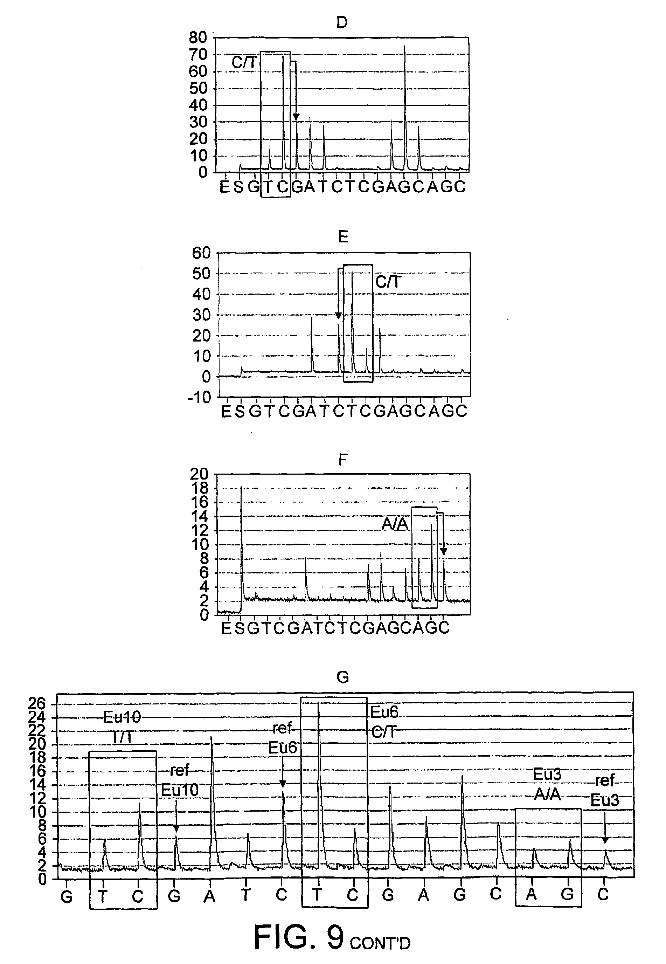

It will be understood that in the method of the invention, differing amounts of nucleic acid template may be present when multiple nucleic acid molecules are to be typed. In order to be able to quantify the number of nucleotides incorporated upon addition in certain embodiments, it is preferred to design the primers and nucleotide dispensation in such a way that a reference signal is generated for each primer which corresponds to a single nucleotide incorporation event. The reference signal is generated in the absence of nucleotide incorporation in the other primer-extension reactions. The reference signal allows for calibration of the signals relating to the same template. The reference peaks are clearly shown on Figure 9, and the height of the variant site signal can be correlated to the reference signal to increase accuracy.

The step of detecting nucleotide incorporation by detecting PPi release results in a signal indicative of the amount of pyrophosphate released, and hence the amount of nucleotide incorporated. In the method of the invention, 2 or more distinct primers are used sequentially or simultaneously in a primer-extension reaction. Thus, in the case of the simultaneously added primers, for every nucleotide addition, 0, 1 or more nucleotides may be incorporated into the growing DNA chains. The signal generated in the pyrophosphate detection step will therefore be indicative of the number of nucleotides incorporated in the primer-extension step for the combination of all primers bound to the template DNA. The size of the signal (i.e. the height of each peak) can therefore be correlated directly to the number of incorporated nucleotides. In certain embodiments, the primer needs only to be subjected to 1 to 20, preferably 1 to 10, e.g. 1 to 5 and most preferably 1 to 4 cycles of nucleotide addition.

In one embodiment of the invention, 2 or more primers are hybridized (simultaneously) at, adjacent or near to variable sites in the target nucleic acid. Each primer being responsible for the typing of one or possibly more variable sites. Primer extension is then performed as described above, and primer extension occurs for each primer only if the nucleotide added is complementary to the target base. Thus, when 2 primers are used simultaneously, none, 1, 2 or more (for homopolymeric regions) nucleotide incorporation events may occur upon the addition of any given nucleotide. The primer extension reaction is carried out simultaneously for all hybridized primers in the reaction mixture. Thus, the detected nucleotide incorporation gives a cumulative picture for all hybridized primers. In this manner, the pattern of nucleotide incorporation may be directly determined. Preferably, when an extension reaction extends across a variable site, nucleotide incorporation occurs only at that site.

In a further particularly preferred embodiment which is also discussed above and in the Examples, the extension primers are hybridized to the template, and the primers are extended simultaneously. The primers are designed to enable primer extension to occur over the variable sites sequentially - i.e. primer extension occurs for each primer simultaneously, but primer extension over a variable site occurs in turn, whilst the other primers are extended over a conserved/semiconserved region or more preferably are not extended at all due to the addition of non-complementary nucleotides. The pattern of nucleotide addition is preferably pre-determined to allow extension of the primers to occur sequentially over the variable sites. The primers may bind 1 to 40, 1 to 20, 1 to 10, 1 to 5 nucleotides from or adjacent to the variable site.

Optionally once a primer has been extended over a variable site, a chain terminator, such as a dideoxynucleotide, may be added to specifically terminate the chain extension reaction of that primer. It will be understood that nucleotide incorporation signals will be generated for all primers during the primer extension reaction, and will contribute to the pattern obtained. Nevertheless, different regions of the pattern will preferably relate to just one of the variable sites.

In a still further modified embodiment of the invention, chain terminators may be employed in place of dNTPs or in combination with dNTPs, using simultaneously hybridised primers. In this case, the primers are selected or designed to ensure that primer extension from each primer takes place sequentially, i.e. that nucleotides are first incorporated from the first primers, the first extension reaction is complete, before nucleotide incorporation from the next primer takes place. This embodiment also requires that the nucleotides are added in predetermined order.

Indeed, so-called "intelligent" primer design may be used to carry out the method of the invention in a desired or pre-selected (i.e. predetermined) manner. This may be applied both to the number of extension primers employed, and to the design of the sequence thereof. "Intelligent" primer design is optimally performed with an "intelligent" order of addition of nucleotides to enable the sequencing of the individual variable sites to be performed in isolation. Such 'intelligent' design of primers and the order of nucleotide addition is described in more detail in the Examples.

The method of the invention may conveniently be performed in a single reaction vessel, Thus, for example, all extension primers may be added together into a single reaction vessel.

In order for the primer-extension reaction to be performed, the nucleic acid molecule, regardless of whether or not it has been amplified, is conveniently provided in a single-stranded format. The nucleic acid may be subjected to strand separation by any suitable technique known in the art (e.g. Sambrook et al., supra), for example by heating the nucleic acid, or by heating in the presence of a chemical denaturant such as formamide, urea or formaldehyde, or by use of alkali.

However, this is not absolutely necessary and a double-stranded nucleic acid molecule may be used as template, e.g. with a suitable polymerase having strand displacement activity.

Where a preliminary amplification step is used, regardless of how the nucleic acid has been amplified, all components of the amplification reaction need to be removed, to obtain pure nucleic acid, prior to carrying out the typing assay of the invention. For example, unincorporated nucleotides, PCR primers, and salt from a PCR reaction need to be removed. Methods for purifying nucleic aids are well known in the art (Sambrook et al., supra), however a preferred method is to immobilize the nucleic acid molecule, removing the impurities via washing and/or sedimentation techniques.

Optionally, therefore, the target nucleic acid may be provided with a means for immobilization, which may be introduced during amplification, either through the nucleotide bases or the primer/s used to produce the amplified nucleic acid.

To facilitate immobilization, the amplification primers used according to the invention may carry a means for immobilization either directly or indirectly. Thus, for example the primers may carry sequences which are complementary to sequences which can be attached directly or indirectly to an immobilizing support or may carry a moiety suitable for direct or indirect attachment to an immobilizing support through a binding partner.

Numerous suitable supports for immobilization of DNA and methods of attaching nucleotides to them, are well known in the art and widely described in the literature. Thus for example, supports in the form of microtitre wells, tubes, dipsticks, particles, fibres or capillaries may be used, made for example of agarose, cellulose, alginate, teflon, latex or polystyrene. Advantageously, the support may comprise magnetic particles e.g. the superparamagnetic beads produced by Dynal AS (Oslo, Norway) and sold under the trademark DYNABEADS. Chips may be used as solid supports to provide miniature experimental systems as described for example in Nilsson et al. (Anal. Biochem. (1995), 224:400-408).

The solid support may carry functional groups such as hydroxyl, carboxyl, aldehyde or amino groups for the attachment of the primer or capture oligonucleotide. These may in general be provided by treating the support to provide a surface coating of a polymer carrying one of such functional groups, e.g. polyurethane together with a polyglycol to provide hydroxyl groups, or a cellulose derivative to provide hydroxyl groups, a polymer or copolymer of acrylic acid or methacrylic acid to provide carboxyl groups or an amino alkylated polymer to provide amino groups. US patent No. 4,654,267 describes the introduction of many such surface coatings.

Alternatively, the support may carry other moieties for attachment, such as avidin or streptavidin (binding to biotin on the nucleotide sequence), DNA binding proteins (e.g. the lac I repressor protein binding to a lac operator sequence which may be present in the primer or oligonucleotide), or antibodies or antibody fragments (binding to haptens e.g. digoxigenin on the nucleotide sequence). The streptavidin/biotin binding system is very commonly used in molecular biology, due to the relative ease with which biotin can be incorporated within nucleotide sequences, and indeed the commercial availability of biotin-labelled nucleotides. This represents one preferred method for immobilisation of target nucleic acid molecules according to the present invention. Streptavidin-coated DYNABEADS are commercially available from Dynal AS.

As mentioned above, immobilization may conveniently take place after amplification. To facilitate post amplification immobilisation, one or both of the amplification primers are provided with means for immobilization. Such means may comprise as discussed above, one of a pair of binding partners, which binds to the corresponding binding partner carried on the support. Suitable means for immobilization thus include biotin, haptens, or DNA sequences (such as the lac operator) binding to DNA binding proteins.

When immobilization of the amplification products is not performed, the products of the amplification reaction may simply be separated by for example, taking them up in a formamide solution (denaturing solution) and separating the products, for example by electrophoresis or by analysis using chip technology. Immobilization provides a ready and simple way to generate a single-stranded template for the extension reaction. As an alternative to immobilization, other methods may be used, for example asymmetric PCR, exonuclease protocols or quick denaturation/annealing protocols on double stranded templates may be used to generate single stranded DNA. Such techniques are well known in the art.

The method of the invention allows the typing (e.g. genotyping) of one or more nucleic acid molecule derived from an individual (e.g. a patient under clinical test, a tissue sample for typing, or a microorganism for identification). Thus, the method of the invention is capable of distinguishing between different genotypes within a species. This is particularly useful in the field of identification of microbial species, where many genotypes of one microbe may exist, for example, there are currently seven known genotypes of the Hepatitis C Virus.

The method of the present invention is particularly advantageous in the diagnosis of pathological conditions characterised by the presence of specific DNA, particularly latent infectious diseases such as viral infection e.g. by herpes, hepatitis or HIV. Also, the method can be used to characterise or type and quantify bacterial, protozoal and fungal infections where samples of an injecting organism may be difficult to obtain or where an isolated organism is difficult to grow *in vitro* for subsequent characterisation as in the case of P. *falciparum* or *Chlamydia* species. Due to the simplicity and speed of the method it may also be used to detect other pathological agents which cause diseases such as syphilis and meningitis. Even in cases where samples of the injecting organism may be easily obtained, the speed of this method compared with overnight incubation of a culture may make the method according to the invention preferable over conventional techniques.

The method of the present invention may be used to analyse two or more single nucleotide polymorphisms (SNPs) within one or more genes, or two or more genes, in an individual. Many diseases and conditions may be associated with (or linked to) combinatorial polymorphisms within the same gene, or within distinct genes. For example, in WO 00/22166, it has been suggested that a combination of SNPs within several genes gives a polymorphic pattern which may be used to predict the likelihood of cardiovascular disease, allowing detailed prognosis for an individual, and predicting whether a particular therapeutic regime would be effective in improving a cardiovascular condition. Thus, the method of the invention can be used to give a quick prognosis on the particular genotype of an individual, allowing tailored therapy to be administered. Example 2 shows that multiplex genotyping can be performed for SNPs in the RAAS system. In this example, one nucleic acid contains 2 SNPs (EU7) and two additional nucleic acids contain 1 SNP each (EU8 and EU11).

The method of the invention is advantageous in that it determines the exact sequence of the variable sites (i.e. is based on a sequencing procedure, it avoids costly and cumbersome procedures, such as electrophoresis, and advantageously labelled nucleotides and/or primers, and large numbers of samples can be analysed in a short time.

The primer extension reaction generates a "pattern" or "fingerprint" indicative of nucleotide incorporation, correlated to the nucleotide added to the reaction mixture. The pattern is a cumulative picture of nucleotide incorporation for the primers designed to detect nucleotide incorporation at 2 or more variable sites within the target nucleic acid molecule(s). To enable the target nucleic acid molecule(s) to be typed, reference patterns are used, using the same variable sites and extension primers. Each genotype should produce a different pattern, facilitating identification by comparison to the reference pattern which can be determined theoretically.

The method of the invention relies upon the knowledge of the location and nature of the variable sites, together with further known sequence information (e.g. with known sequences of conserved/semi-conserved regions) from which to determine an appropriate primer binding site and design a complementary extension primer. Using the method of the invention, any combination of variable sites may be used in the typing method. It will be understood by those skilled in the art that the method of the invention is not limited to multiple variable sites within genes, but the method is also applicable to non-coding regions. The pattern may be obtained for variable sites which are in one or more of the same gene, in related genes, in disparate genes, or in non-coding regions.

The invention also comprises kits for carrying out the method of the invention, particularly for typing one or more nucleic acid molecules containing one or more variable sites. These will normally include one or more of the following components:
(a) optionally primer(s) for *in vitro* amplification; (b) two or more primers for the primer extension reaction each primer binding at a different predetermined site in a nucleic acid molecule, and said primers being designed to enable, when said primers are simultaneously hybridized, primer extension to occur over said variable sites sequentially, upon the addition of nucleotides in a pre-determined manner; (c) nucleotides for amplification and/or for the primer extension reaction (as described above); (d) a polymerase enzyme for the amplification and/or primer extension reaction; and (e) means for detecting primer extension by sequencing-by-synthesis (e.g. means of detecting the release of pyrophosphate as outlined and defined above).

In certain embodiments, the kit will also include instructions for the order of addition of the nucleotides.

The invention will now be described by way of nonlimiting examples with reference to the drawings in which:-
Figure 1 shows schematically one method for the typing of nucleic acid using multiple primers (multiplexing) simultaneously in a primer extension reaction;
Figure 2 shows the sequence of the 5' untranslated region (5'-UTR) of seven Hepatitis C virus (HCV) genotypes, wherein the arrows indicate the positions of the amplification and extension primers, and the nucleotides highlighted in bold type illustrate the variable region to be sequenced by the primer extension reaction;

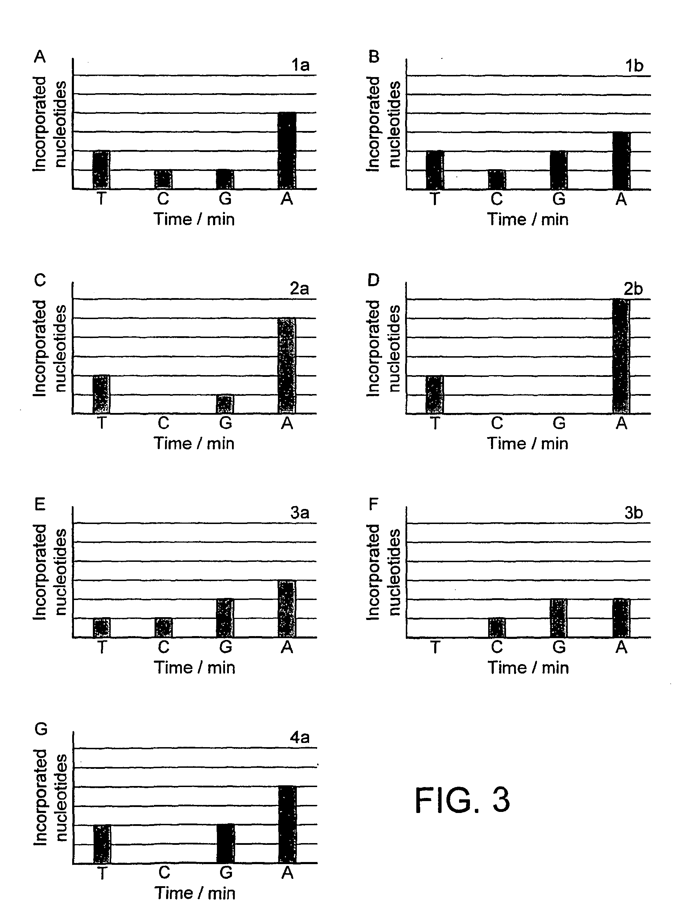

Figure 3 shows theoretical traces which would be obtained (light generated (indicating nucleotide incorporation) versus time (and nucleotide addition)), for the seven genotypes of HCV studied. The experimental conditions and extension primers theoretically used are described in Example 1. Three distinct extension primers were theoretically used simultaneously in a primer-extension reaction mixture. Inorganic pyrophosphate PPi is released in a DNA-polymerase catalyzed reaction if a nucleotide is incorporated. The PPi is monitored by coupled enzymatic reactions using ATP sulphurylase and luciferase. Light generated as a result is measured by a CCD detector or luminometer;
Figure 4 shows traces (light generated (indicating nucleotide incorporation) versus time (and nucleotide addition)), obtained for six samples containing different HCV genotypes. The experimental conditions and primers used are described in Example 1. The incorporation of a nucleotide into the extending primer results in the release of PPi, which is detected using a coupled enzymatic reactions using ATP sulphurylase and luciferase. Light generated as a result of successful extension is measured by a CCD camera or luminometer;
Figure 4a shows the trace obtained for HCV genotype 1a;
Figure 4b shows the trace obtained for HCV genotype 1b;
Figure 4c shows the trace obtained for HCV genotype 2a;
Figure 4d shows the trace obtained for HCV genotype 2b;
Figure 4e shows the trace obtained for HCV genotype 3a;
Figure 4f shows the trace obtained for HCV genotype 3b;
Figure 5 shows three potential primer binding positions for the SNP Eu6 from the ACE gene (Angiotensin Converting Enzyme). Figure 5a shows a primer (boxed nucleotides) bound to the template with its 3' end 4 nucleotides from the SNP position, Figure 5b shows a primer bound to the template with the 3' and 5 nucleotides from the SNP position and in Figure 5c the primer is bound 10 nucleotides from the SNP position. In all figures, the 2 potential variants at the SNP site are shown (G/A in template strand);
Figure 6 shows the theoretical output from Pyrosequencing^{™} reactions for two SNP positions. The theoretical output is plotted as nucleotide dispensed into the reaction versus peak height (correlated to light emitted from the Pyrosequencing^{™} reaction). Figure 6a shows the theoretical output for sequencing G/ACAG, in this case, the primer would be adjacent to the polymorphic position. The theoretical output shown is for the heterozygote (i.e. the individual has one copy of the SNP A and one copy of the SNP G). Figure 6b shows the theoretical output for TGAAC/TA. The primer is thus bound 4 nucleotides away from the SNP. Again, the pattern shown would result from a heterozygous individual (C/T). Figure 6c shows a cumulation of the two individual sequencing reactions in one primer extension reaction mixture;.
Figure 7 shows a simplified multiplexing analysis wherein the extension primers are designed in such a way that their 3' ends are positioned at different distances from the polymorphic position. This enables the design of an "intelligent" order of addition of nucleotides to be determined to enable the SNP (marked X) to be sequenced in isolation. Thus, the extension primers should be designed in parallel with the dispensation order;
Figure 8 shows the theoretical output for five SNPs present in the RAAS system - Eu4 (ACE G2215A), Eu8 (ATG C521T), Eu10 (ATP T573C), Eu6 (ACE T3409C) and Eu3 (ACE T1237C). The theoretical outputs are plotted as in Figure 7. The extension primers are positioned such that the sequence that is analysed for Eu4 is G/A CTGCCTG, Eu8 is CACCA/G TGG, Eu10 is C/TCCGATAGGGC, Eu6 is ACTTC/TG and Eu3 is AGACA/GGGC;
Figures 8a and 8b show the theoretical output expected to be obtained when the SNPs Eu4 and Eu8 are typed in a standard one-primer only reaction. The SNP nucleotide incorporation position are framed;

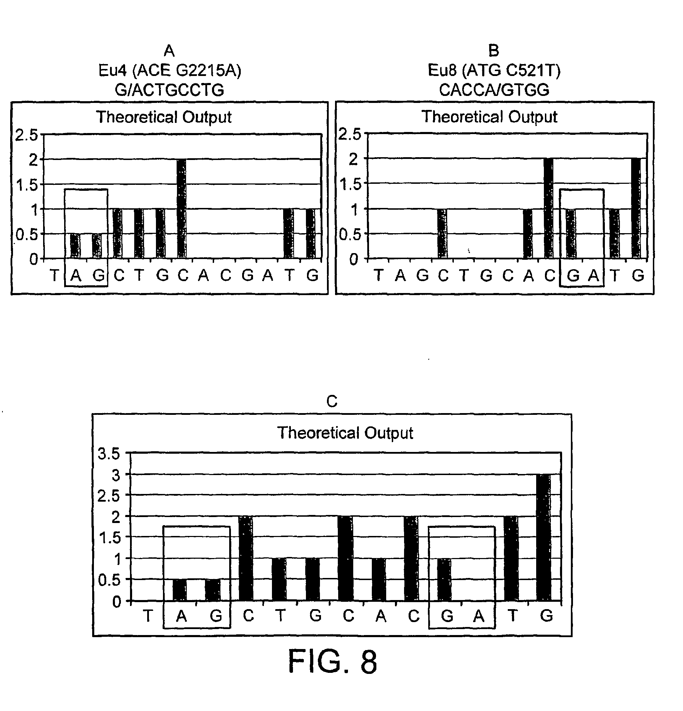

Figure 8a shows the theoretical output when the individual is heterozygous (A/G) and Figure 8b shows the output expected when the individual is homozygous (G/G).
Figure 8c shows the output expected when the two SNPs are sequenced simultaneously in the same reaction (multiplexed). The polymorphic positions are framed;

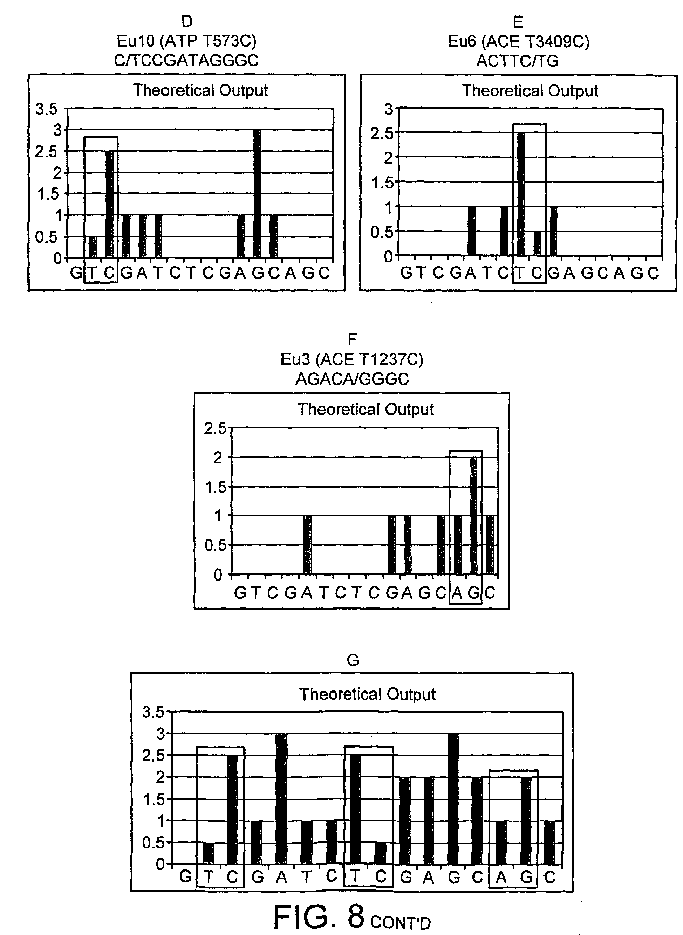

Figures 8d, 8e and 8f are the theoretical results obtained from sequencing Eu10, Eu6 and Eu3 alone, respectively. SNP Eu10 and Eu6 are shown as heterozygotes (C/T and C/T, respectively) and Eu3 as a homozygote (A/A). The theoretical patterns for the 3 SNPs are combined in Figure 8g, and the SNP positions are framed;
Figure 9 shows the results obtained as traces (light generated (indicating nucleotide incorporation) versus time and nucleotide incorporation for seven reactions containing differing templates and primer combinations. The experimental conditions and primers used are described in Example 2. The incorporation of a nucleotide into the extending primer results in the release of PPi, which is detected using a coupled enzymatic reactions using ATP sulphurylase and luciferase. Light generated as a result of successful extension is measured by a CCD camera or luminometer;
Figure 9a shows the trace obtained for Eu4;
Figure 9b shows the trace obtained for Eu8;
Figure 9c shows the trace obtained for Eu4 and Eu8 simultaneously sequenced;
Figure 9d shows the trace obtained for Eu10;
Figure 9e shows the trace obtained for Eu6;
Figure 9f shows the trace obtained for Eu3;
Figure 9f shows the trace obtained for Eu10, Eu6 and Eu3, simultaneously sequenced;

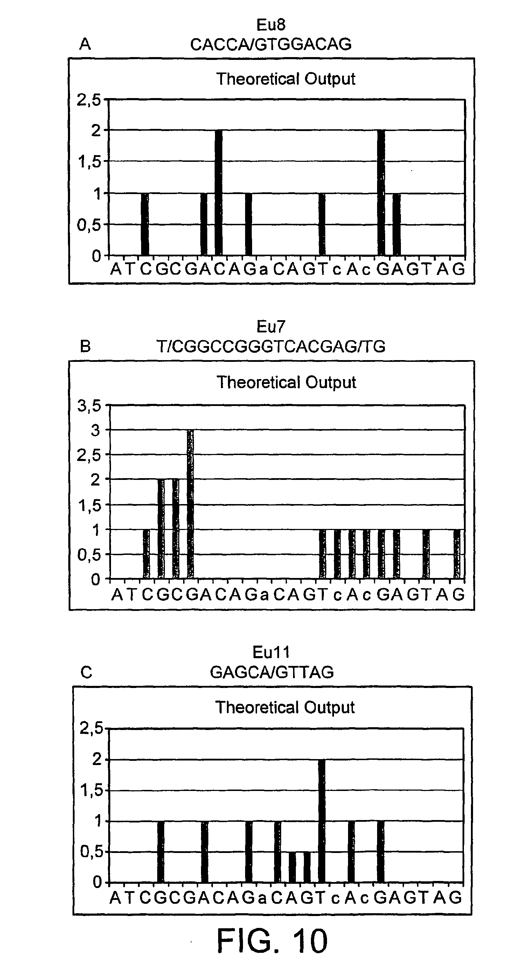

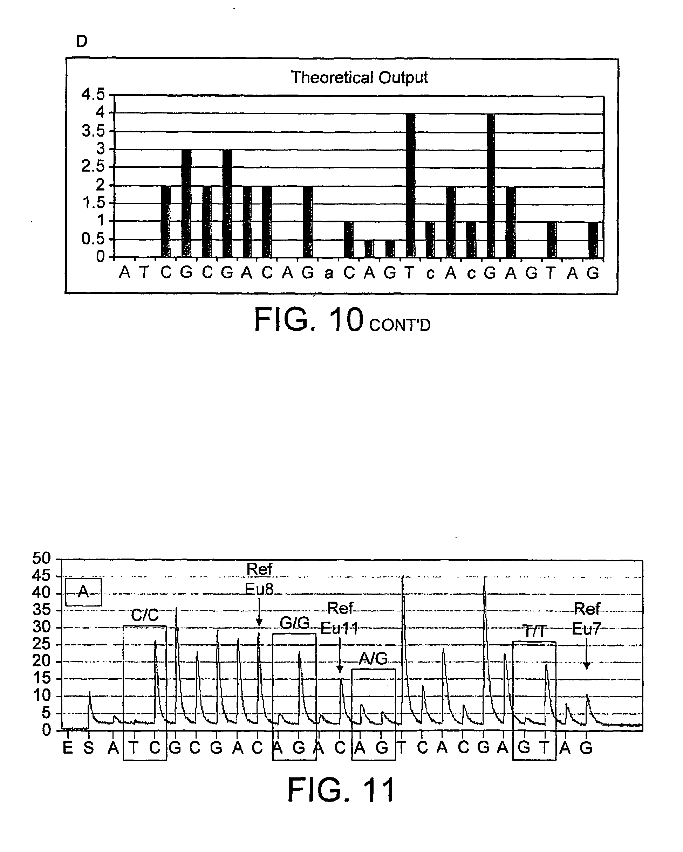

Figure 10 shows the theoretical output for SNPs Eu8, Eu7 and Eu11 present in the RAAS system. The theoretical outputs are plotted as in Figure 7. The sequences analyzed are Eu8 CACCA/GTGGACAG, Eu7 T/CGGCCGGGTCACGAG/TG and Eu11 GAGCA/GTTAG. Therefore, the fragment Eu7 contains two polymorphic sites;
Figure 10a shows the theoretical trace for Eu8 (G/G) ;
Figure *10b shows the theoretical trace for Eu7 (C/C and T/T) ;
Figure 10c shows the theoretical trace for Eu11 (A/G); and
Figure 10d shows the multiplex theoretical trace for Eu7, Eu8 and Eu11;
Figure 11 shows the result obtained as a trace (light generated versus nucleotide addition) for the multiplex reaction as defined in Example 3. The polymorphic positions are framed and the reference peaks shown (arrows). The genotype for the individual typed is Eu8 G/G, Eu7 C/C and T/T Eu11 A/G;

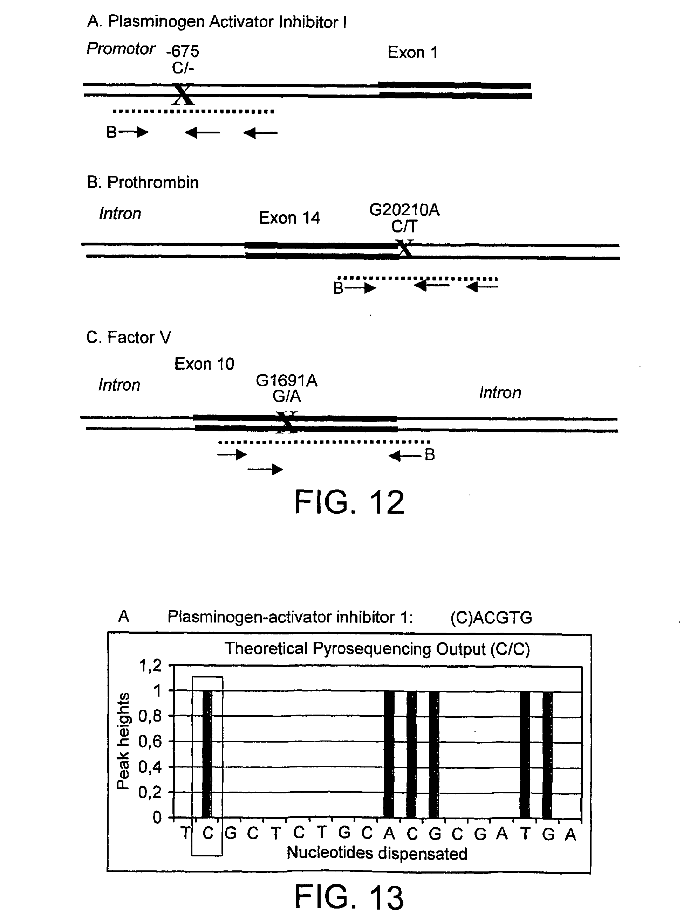

Figure 12 is a scheme showing primer design for 3 separate nucleic acid fragments, for the Plasminogen Activator Inhibitor I gene, the Prothrombin gene and the Factor V gene. The arrows marked with a * correspond to the sequencing primers and the outer arrows correspond to the PCR primers. The biotinylated primer is indicated with a B. The polymorphic position in the gene of interest is marked by an X;

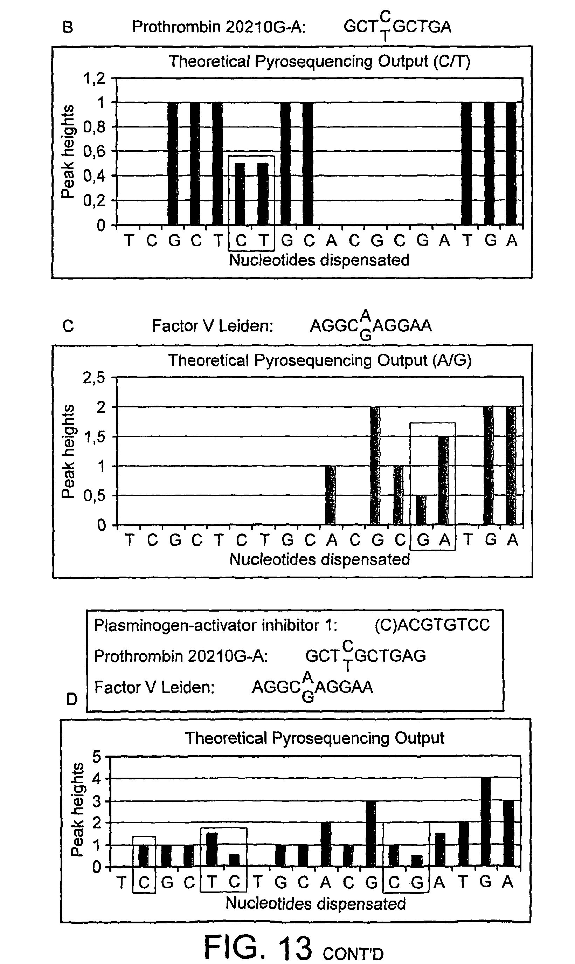

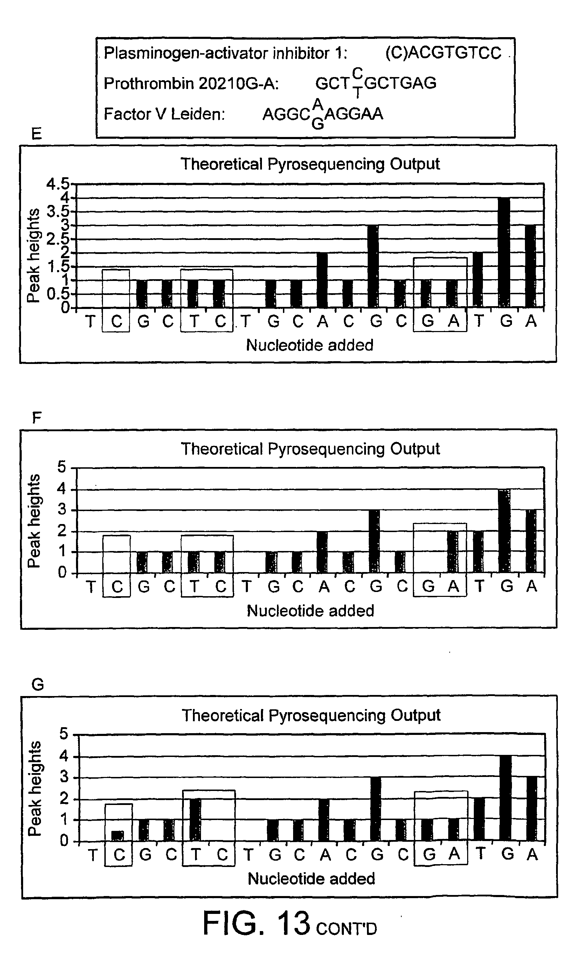

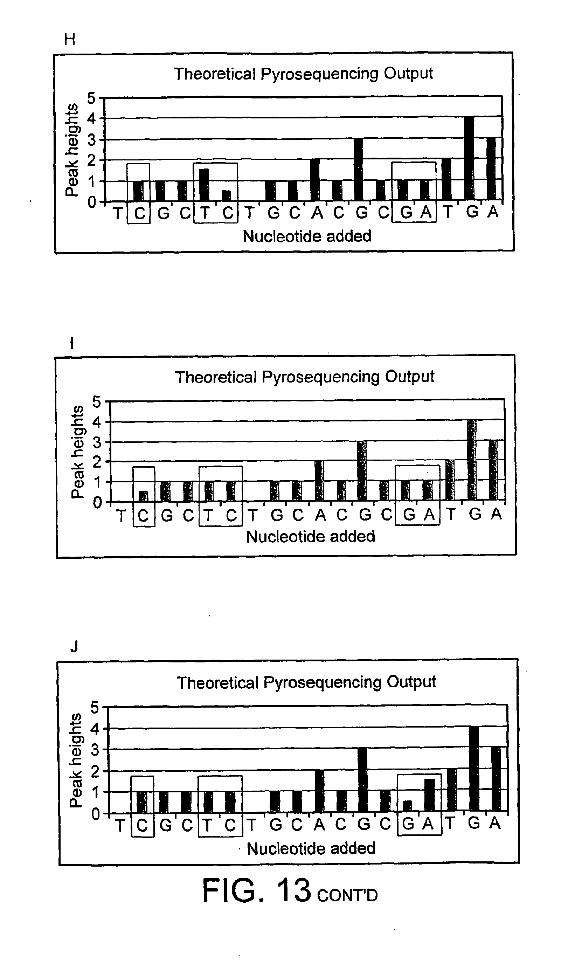

Figure 13 shows the expected output from Pyrosequencing^{™} reactions for SNPs in Plasminogen-activator inhibitor 1 (4G/5G deletion), Prothrombin (G20210A) and Factor V (G1691A). The theoretical output is shown as for Figure 7. The sequence analysed for PAI1 is (C)ACGTG, Prothrombin is GCTC/TGCTGA and Factor V AGGCA/GAGGAA;
Figure 13a shows the theoretical trace for PAI1 (C/C - no deletion);
Figure 13b shows the theoretical trace for Prothrombin (C/T);
Figure 13c shows the theoretical trace for Factor V (A/G);
Figure 13d shows the theoretical trace for a combination of the three SNPs in one multiplex reaction;
Figure 13e shows the theoretical trace for the genotype PAI1 C/C (no deletion), Prothrombin C/C and Factor V G/G;
Figure 13f shows the theoretical trace for the genotype PAI1 double deletion, Prothrombin C/C and Factor V A/A;
Figure 13g shows the theoretical trace for the genotype PAI1 del/C, Prothrombin T/T and Factor V G/G;
Figure 13h shows the theoretical trace for the genotype PAI1 C/C (no deletion), Prothrombin T/C and Factor V G/G;
Figure 13i shows the theoretical trace for the genotype PAI1 C/del, Prothrombin C/C and Factor V G/G; and
Figure 13j shows the theoretical trace for the genotype PAI1 C/C (no deletion), Prothrombin C/C and Factor V A/G;

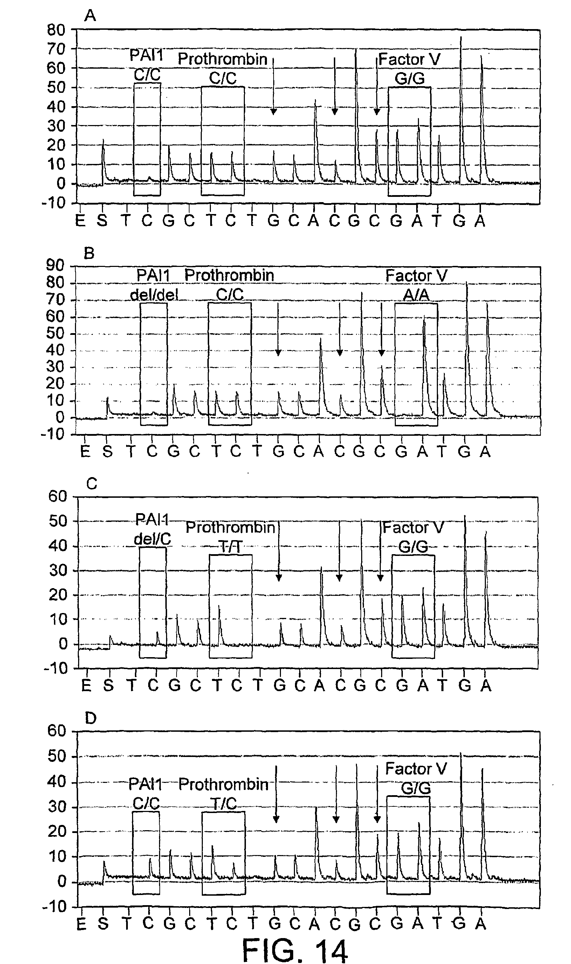

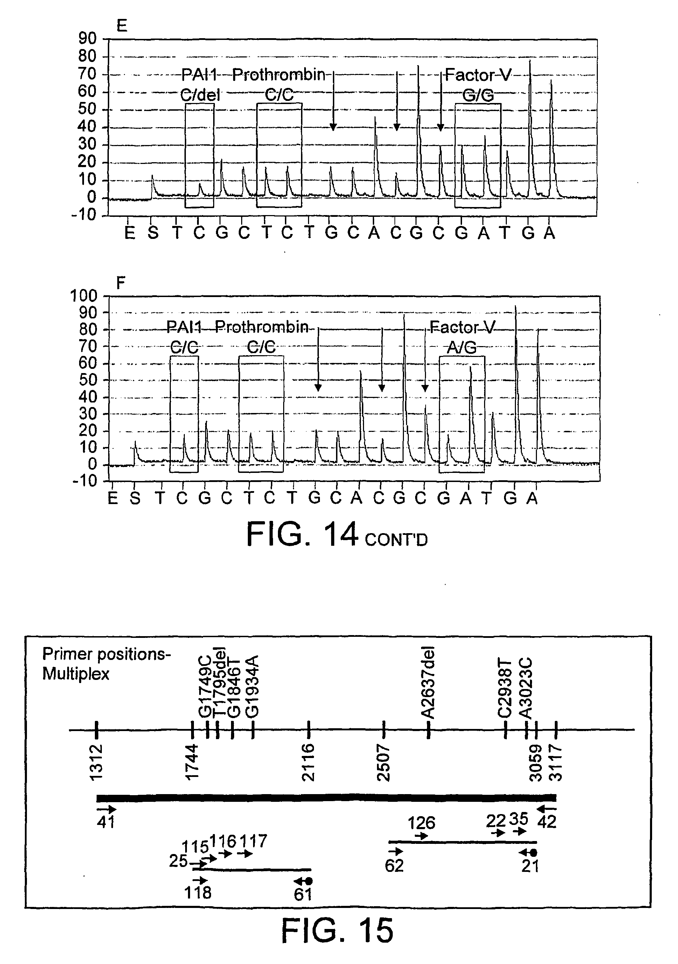

Figure 14 shows the results obtained as traces (light geneated versus nucleotide addition) for six reactions. The materials and methods are described in Example 4;
Figure 14a shows the trace obtained for the genotype PAI1 C/C (no deletion), Prothrombin C/C and Factor V G/G and corresponds to the theoretical pattern shown on Figure 13e;
Figure 14b shows the trace obtained for the genotype PAI1 double deletion, Prothrombin C/C and Factor V A/A and corresponds to the theoretical pattern shown on Figure 13f;
Figure 14c shows the trace obtained for the genotype PAI1 del/C, Prothrombin T/T and Factor V G/G and corresponds to the theoretical pattern shown on Figure 13g;
Figure 14d shows the trace obtained for the genotype PAI1 C/C (no deletion), Prothrombin T/C and Factor V G/G and corresponds to the theoretical pattern shown on Figure 13h;
Figure 14e shows the trace obtained for the genotype PAI1 C/del, Prothrombin C/C and Factor V G/G and corresponds to the theoretical pattern shown on Figure 13i; and
Figure 14f shows the trace obtained for the genotype PAI1 C/C (no deletion), Prothrombin C/C and Factor V A/G and corresponds to the theoretical pattern shown on Figure 13j;
Figure 15 depicts the localisation of the primers with regard to the CYP2D6 gene. A segment of the gene with particular highlighted polymorphisms can be seen at the top of this figure. The 61118 and 2162 fragments, as amplified by nested PCR primers are at the bottom of the figure. The extension primers used for the multiplexing reactions are shown above the 61118 and 2162 fragments;

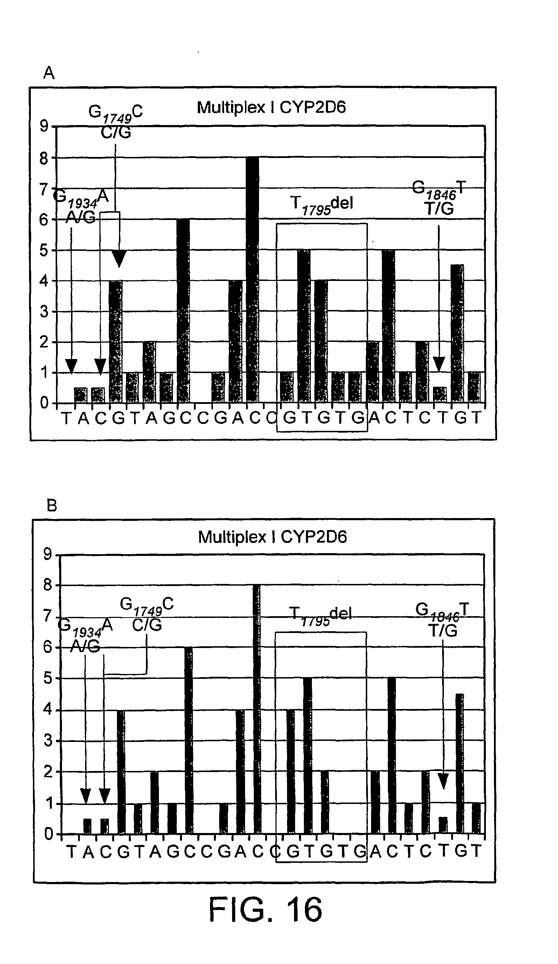

Figure 16 represents the theoretical output obtained for two genotypes of the CYP2D6 gene. The traces were calculated as described previously;
Figure 16a shows the theoretical output for G1934 A (A/G), G 1749 C (C/G), T1795 del (no deletion) and G 1846 T (T/g);
Figure 16b shows the theoretical output for G1934 A (A/G), G 1749 C (C/G), T1795 del T/deletion and G1846T (T/G); and

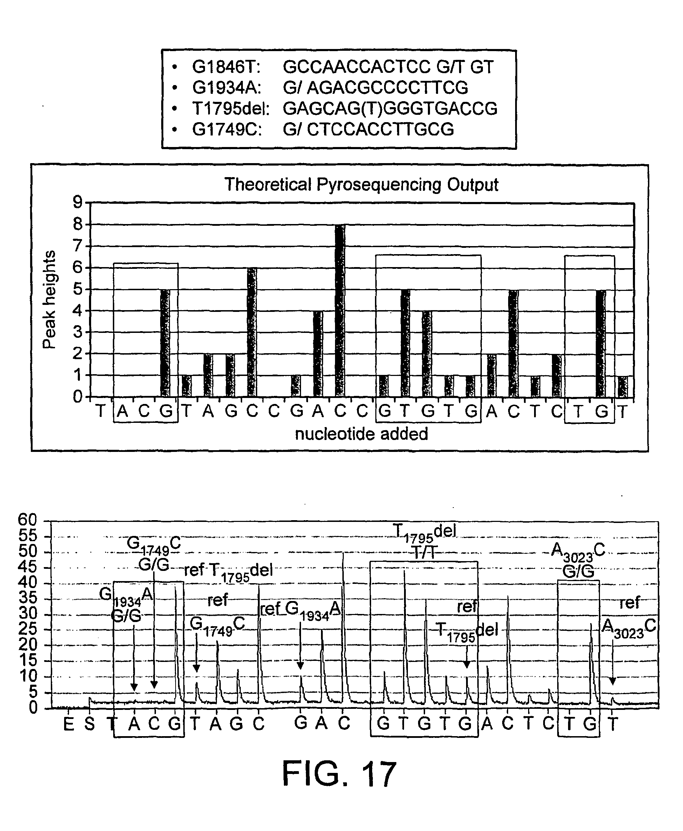

Figure 17 shows the result obtained from Example 5 as a trace (light generated versus nucleotide added). The experimental conditions are described in Example 5. The genotype of the individual typed is G1934A G/G, G1749C G/G, T1795 del T/T (no deletion) G1846T G/G. Also shown is the theoretical output plot for this genotype.

### EXAMPLE 1

### Serum samples

72 sera from HCV-positive Veterans were obtained from Stanford Veteran hospital. 10 HCV-positive sera were obtained from Iran.

### Synthesis and purification of oligonucleotides

The oligonucleotides HCV-PCR-OUTF
(5'- CCCTGTGAGGAACTWCTGTCTTCACGC), HCV-PCR-OUTR
(5'-GCTCATGRTGCACGGTCTACGAGACCT), HCV-PCR-INF
(5'-TCTAGCCATGGCGTTAGTAYGAGTGT), BHCV-PCR-INR
(5'-Biotin-CACTCGCAAGCACCCTATCAGGCAGT), HCV-SEQF1
(5'-GGAACCGGTGAGTACACCGGAAT), HCV-SEQF2
(5'-GACYGGGTCCTTTCTTGGA), HCV-SEQF3
(5'-ATTTGGGCGTGCCCCCGC), were all synthesized and HPLC purified by MWG Biotech (High points, NC, USA).

### RNA extraction, cDNA synthesis and amplification

RNA was extracted from 100 µl of patient sera using Ambion's Totally RNA isolation kit (www.ambion.com, Ambion (Europe) Ltd., Cambridge, UK). cDNA was synthesized using the kit Superscipt^{™} Preamplification system from Invitrogen (www.invitrogen.com, Invitrogen Ltd., Paisley, UK). First strand cDNA synthesis employed an RNA/primer mixture containing, 5 µl RNA and 1 µl 0.5 µg/µl Oligo (dT) random primer which was incubated at 70°C for 10 min and then placed on ice for at least 1 min. A reaction mixture contating 2 µl 10 X PCR buffer (200mM Tris-HCl (pH 8.4), 500 mM KCl 2 µl 25 mM MgCl₂ 10 mM dNTP mix and 0.1 M DTT, was added to each RNA/primer mixture, mixed gently collected by brief centrifugation and then incubated at 42°C for 5 min. Two hundred units of Superscript II Reverse Transcriptase was added to each tube, and incubated at 40°C for 50 min. The reaction was terminated by incubating at 70°C for 15 minutes and then chilled on ice. The nucleic acid was collected by brief centrifugation. 1 µl of RNase H was added to each tube and incubated for 20 mm at 37°C. Outer PCR was performed on 1 µl of cDNA using HCV-PCR-OUTF and HCV-PCR-OUTR PCR. The outer PCR was diluted by 500,000 times and 1 µl of that was used as a template for inner PCR using primers HCV-PCR-INF and HCV-PCR-INR.

### Template Preparation

The biotinylated PCR products were immobilized onto streptavidin-coated super paramagnetic beads Dynabeads^{™} M280-Streptavidin (Dynal Biotech ASA, Oslo, Norway). Single-stranded DNA was obtained by discarding the supernatant after incubation of the immobilized PCR product in 0.10 M NaOH for 3 min. Five pmol of sequencing primers HCV-SEQF1, HCV-SEQF2, and HCV-SEQF3 were hybridized to the immobilized strand, as described in Ronaghi et al., 1996, Analytical Biochemistry, 242, 84-89.

### Primer extension reaction

The primed DNA templates were placed in a microtiter plate containing 0.5 µg SSB (Amersham Pharmacia Biotech, USA), and Pyrosequencing^{™} substrates and enzymes (www.pyrosequencing.com Pyrosequencing AB, Uppsala, Sweden) nucleotides were dispensed using fully automated microtiter plate-based PSQ^{™} Pyrosequencing^{™} instrument. The sequencing procedure was carried out by stepwise elongation of the primer-strand upon pre-specified addition of four different nucleotides. The template was hybridized with the three extension primers described above. The progress of sequencing was followed in real-time using Pyrosequencing^{™} Tag software, (Pyrosequencing^{™} AB, Uppsala, Sweden) and subtyping was performed manually.

HCV positive blood sera from 89 different patients was collected and HCV RNA was extracted as described above. Subsequent to cDNA synthesis, PCR was performed to amplify a 236-base long region from 5' UR. One of the primers in the PCR was biotinylated. After capture of the PCR products on magnetic beads and template preparation, sequencing-by-synthesis was performed.

### Results

### Principle of the HCV typing method.

The principle of the typing method described above is outlined in Figure 1. In this model system, extension primers are hybridized to the target sample DNA, which is immobilized on magnetic beads.

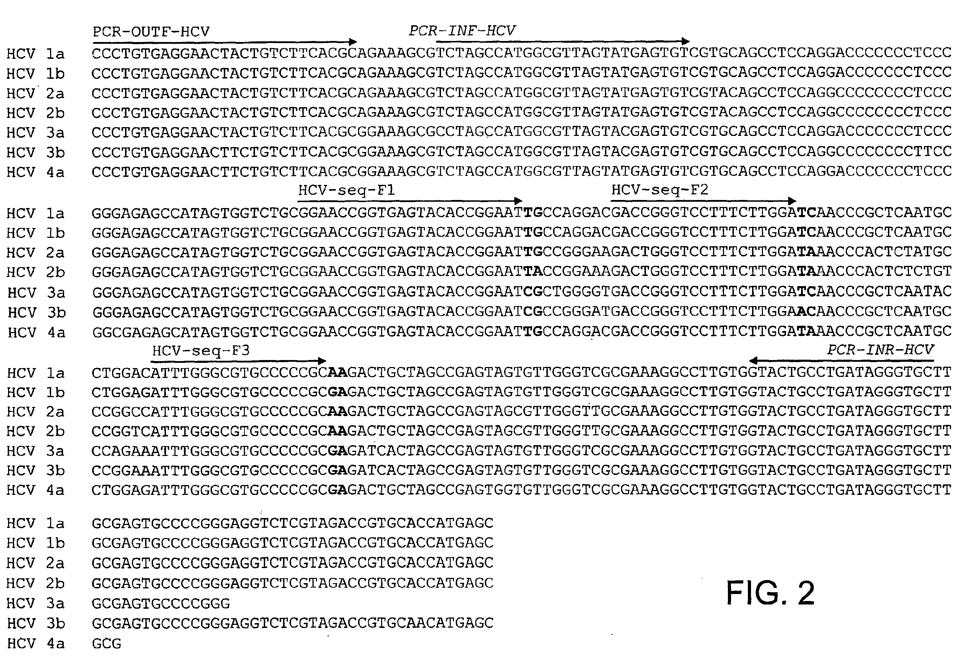

The extension primers hybridise specifically to the conserved region adjacent to the variable region. In this set of experiments, 3 sequencing primers for HCV were used. The primers and their alignment to the HCV genomes are shown in Figure 2.

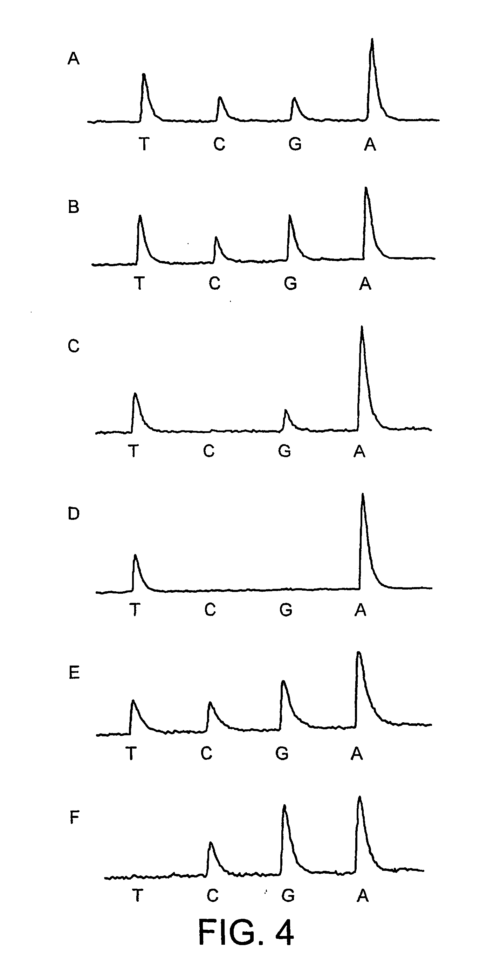

The signals resulting from the specific extension of each primer are directly correlated to the number of nucleotides incorporated. The 'fingerprint' produced can therefore be used to identify the genotype of the individual, against reference fingerprints, which can be theoretically deduced from the sequences of the variable regions. References fingerprints calculated theoretically from the sequence of the variable regions are shown on Figure 3. These can be used to type the results shown on Figure 4: Figure 4a is the fingerprint for HCV 1a, Figure 4b is the fingerprint for HCV 1b, Figure 4c is the fingerprint for HCV 2a, Figure 4d is the fingerprint for HCV 2b, Figure 4e is the fingerprint for HCV 3a, and Figure 4f is the fingerprint for HCV 3b. Therefore, using the method of the invention, it was possible to genotype HCV infection. Of the 77 sera analyzed by the method of the invention. 35% were infected with HCV 1a, 29% with HCV 1b, 21% with HCV 2a, 4% with HCV 2b, 1% with HCV 3a and 10% with HCV 3b. Of the 10 analysed samples from Iran, the following results were obtained; 1a, 1; 1b, 3; 2a, 3; 3a, 2 and 3b; 1.

### EXAMPLE 2

### Typing of SNPs in the RAAS System

### Templates and Primers

Genomic DNA was isolated according to standard methods, PCR templates was generated with specific primers according to the table below.

| | | | |
|---|---|---|---|
| PCR fragment | PCR primer 1 5'-biotin | PCR primer 2 | From ref. US 6197505 |
| Eu3 | GGA CCA GCT CTC CAC AGT GC | GCC AGC ACG TCC CCA AT | ACEe8R (PCR2) |
| Eu4 | GAT TCC CCT CTC CCT GTA CCT | GCC AGG AAG TTT GAT GTG AAC | ACEe15R (PCR1) |
| Eu6 | CTC GCT CTG CTC CAG GTA C | GCC TCC TTG GAC TGG TAG AT | ACEe24F (PCR2) |
| Eu8 | CCA GGG CAG GGC TGA TA | CAA ACG GCT GCT TCA GGT | ANGe2f3F |
| Eu10 | CAT TTC TTG GTT TGT TCT TCT GA | GTT TGT GCT TTC CAT TAT GAG TC | AT1e5f3F |

The following sequencing primers were used in the multiplex reactions:

| | | |
|---|---|---|
| Eu3 | Eu3s | 5'- CCC CGA CGC AGG GAG AC-3' |
| | A062RS | 5'-CCC CGA CGC AGG GAG-3' |
| | A0943S | 5'- CCC CGA CGC AGG G 3' |
| Eu4 | Eu4s | 5'- GAC CTA GAA CGG GCA GC - 3' |
| | A097FS | 5'- GTT CAG GAC CTA GAA - 3' |
| Eu6 | Eu6s | 5'- CCT CGC TCC GCT CCA GGT A-3' |
| | A091FS | 5'-CTC GCT CTG CTC-3' |
| | A063FS | 5'-CTC GCT CTG CTC CAG GT-3' |
| Eu8 | A089RS (Eu8s) | 5' - GCT GTG AAC ACG CCC AC-3' |
| | A060FS | 5'-GCT GCT GCT GCT CA-3' |
| Eu10 | A088FS (Eu10s) | 5' - AGA TCC CAA AAT TCA ACC CT-3' |

### PCR Amplification

The target nucleic acid molecules were amplified by PCR, either by standard PCR or by multiplex PCR.

Simplex PCR: A 50 µl PCR reaction was set up for each SNP-specific fragment and sample. All fragments were amplified with the AmpliTaq Gold kit (PE Biosystems) and 1.5 mM MgCl₂ according to the following protocol. (Table 1) .

**Table 1.**

| **PCRmix** | **1x** | **100x** |
|---|---|---|
| 10xPCRbuffer | 5 | 500 |
| MgCl₂ (25 mM) | 3 | 300 |
| dNTP (2.5 mM) | 2.5 | 250 |
| DMSO | 0 | 0 |
| Primer a (10 µM) | 1 | 100 |
| Primer b (10 µM) | 1 | 100 |
| TaqGold (5 units/µl) | 0.3 | 30 |
| H₂O | 32.2 | 3220 |
| Sum: | 45 | 4500 |

5 µl genomic DNA (2ng/µl) was added to 45 µl PCR mix.

### PCR cycling conditions:

95°C 5 min, 50x (95°C 15s, 57°C 30s, 72°C 45s), 72°C 5 min, 4°C

Multiplex PCR using 4 amplification primers: A 50 µl PCR reaction was set up using Eu4 and Eu8 SNP-specific fragments. All samples were amplified with the HotStarTaq Master Mix Kit from Qiagen adding Q-solution and MgCl₂ to a final concentration of 2.0 mM according to the following protocol (Table 3).

**Table 3. Magnesium concentration 2.0 mM**

| **PCRmix** | **1x** | **100x** |
|---|---|---|
| 10xPCRbuffer (15 mM MgCl₂) | 5 | 500 |
| MgCl₂ (25 mM) | 1 | 100 |
| dNTP (2.5 mM) | 2.5 | 250 |
| Q-solution | 10 | 1000 |
| Primer 4a (10 µM) | 2 | 200 |
| Primer 4b (10 µM) | 2 | 200 |
| Primer 8a (10 µM) | 2 | 200 |
| Primer 8b (10 µM) | 2 | 200 |
| TaqGold (5 units/µl) | 0.25 | 25 |
| H₂O | 13.25 | 1325 |
| Sum: | 40 | 4000 |

10 µl genomic DNA (2ng/µl) was added to 40 µl PCR mix.

### PCR cycling conditions:

95°C 15min, 35x(94°C 30s, 55°C 1 min, 72°C 2 min), 72°C 10 min, 4°C.

Multiplex PCR using 6 amplification primers: A 50 µl PCR reaction was set up using Eu3, Eu6 and Eu10 SNP-specific fragments. All samples were amplified with the HotStarTaq Master Mix Kit from Qiagen adding Q-solution and MgCl₂ to a final concentration of 2.0 mM according to the following protocol (Table 4).

**Table 4. Magnesium concentration 2.0 mM**

| **PCRmix** | **1x** | **100x** |
|---|---|---|
| 10xPCRbuffer | 5 | 500 |
| MgCl₂ (25 mM) | 1 | 100 |
| dNTP (2.5 mM) | 2.5 | 250 |
| Q-solution | 10 | 1000 |
| Primer 3a (10 µM) | 2 | 200 |
| Primer 3b (10 µM) | 2 | 200 |
| Primer 6a (10 µM) | 2 | 200 |
| Primer 6b (10 µM) | 2 | 200 |
| Primer 10a (10 µM) | 2 | 200 |
| Primer 10b (10 µM) | 2 | 200 |
| TaqGold (5 units/µl) | 0.25 | 25 |
| H₂O | 10.25 | 1025 |
| Sum: | 40 | 4500 |

10 µl genomic DNA (2ng/µl) was added to 40 µl PCR mix.

### PCR cycling conditions:

95°C 15min, 35x(94°C 30s, 59°C 1 min, 72°C 2 min), 72°C 10 min, 4°C.

### Sample Preparation

25 µl of PCR product (multiplex PCR product or pooled standard PCR product) was immobilised by the addition of 10 µl Dynabeads^{™} (Dynal Biotech ASA, supra) (10 µg/µl) together with 25 µl 2xBW buffer (10 mM Tris-HCl pH 7.57, 2M NaCl, 1 mM EDTA and 0.1% Tween 20). 15 pmol sequencing primer was added in annealing buffer (20 mM Tris-Acetate pH 7.51, 5 mM MgAc2) and the mixture incubated for 2 minutes at 80°C. The samples were then allowed to cool to room temperature. 2.2 µg SSB (Amersham Pharmacia Biotech, supra) may be added at this point, if required.

### Primer Extension

The primed DNA templates were placed in a microtiter plate containing Pyrosequencing^{™} substrates and enzymes (PSQ96^{™} plate, Pyrosequencing AB, supra). Nucleotides were dispensed using fully automated microtiter-plate based PSQ^{™} Pyrosequencing^{™} instrument. The sequencing procedure was carried out by stepwise elongation of the primer-strand upon pre-specified addition of four different nucleotides. The templates were hybridized with the extension primers mentioned above. The progress of sequencing was followed in real-time using Pyrosequencing^{™} software.

### Results

### Principle of the SNP typing method.

The principle of the typing method described above is outlined in figure seven. In this model system, extension primers are hybridized to the target sample DNA, which is immobilised on magnetic beads.

In this set of experiments 3 sequencing primers for the RAAS system were used, either in isolation to show the 'simplex patterns' or in combination to show the multiplex patterns.

The signals resulting from the specific extension of each primer are directly correlated to the number of nucleotides incorporated. The 'fingerprint' produced can therefore be used to identify the genotype of the individual, against reference fingerprints, which can be theoretically deduced from the sequences of the variable regions. Reference fingerprints calculated theoretically from the sequence of the SNPs are shown on Figure 8. 8a is the theoretical output for SNP Eu4 (ACE G2215A), 8b is the theoretical output for SNP Eu8 (ATG C521T) and 8c is the theoretical output for the simultaneous analysis of SNPs Eu4 and Eu8, the polymorphic positions are framed. 8d is the theoretical output for SNP Eu10 (ATP T573C), 8e is the theoretical output for SNP Eu6 (ACE T3409C), 8f is the theoretical output for Eu3 (ACE T1237C) and 8g is the theoretical output for the simultaneous analysis of SNPs, Eu10, Eu6 and Eu3. SNPs Eu4, Eu10 and Eu6 are shown as heterozygotes, SNPs Eu8 as homozygote G and SNP Eu3 as homozygote A. These "reference" patterns can be used to type the results shown in Figure 9: 9a is the sequencing data for SNP Eu4 (A/G), 9b is the sequencing data for SNP Eu8 (G/G) and 9c is the multiplex sequencing data for the combination of SNP Eu4 (A/G) and SNP Eu8 (G/G), which correlates to theoretical output 8c, the frames indicating SNP positions. 9d, 9e and 9f are the sequencing data plots for SNP Eu10 (C/T), Eu6 (C/T) and Eu3 (A/A), respectively, and 9g is the multiplex sequencing data for the combination of these 3 SNPs, the polymorphic positions are boxed. Pattern 9g correlates to Figure 8g.

### Example 3

Triplex genotyping on 4 SNPs in the RAAS System - Eu7 Eu8 (containing 2 SNPs), and Eu11.

### Templates and Primers

| | | | |
|---|---|---|---|
| PCR | PCR primer 1 | PCR primer 2 | From ref. |
| fragment | 5'-biotin | | US 6197505 |
| Eu7 | TGA TGT AAC CCT CCT CTC CA | CGG CTT ACC TTC TGC TGT | ANPf4F |
| | | AGTA | |
| Eu8 | CCA GGG CAG GGC TGA TA | CAA ACG GCT GCT TCA GGT | ANGe2f3F |
| Eu11 | TTT CTC CTT CAA TTC TGA | GCC CCT CAG ATA ATG TAA | AT1-spec.1 |
| | AAA GTA | GC | |

### Sequencing primers

| | | |
|---|---|---|
| Eu7 | Eu7s | 5'- ACG GCA GCT TCT TCC CC-3' |
| Eu8 | A089RS (Eu8s) | 5'- GCT GTG AAC ACG CCC AC-3' |
| | A060FS | 5'-GCT GCT GCT GCT CA-3' |
| Eu11 | Eu11s | 5'- GCA GCA CTT CAC TAC CAA AT-3' |

PCR Amplification, sample preparation and primer extension reactions were performed as described in Example 2, with the exception of Eu11, which was amplified according to the protocol in Table 2.

**Table 2.**

| **PCRmix** | **1x** | **100x** |
|---|---|---|
| 10xPCRbuffer | 5 | 500 |
| MgCl₂ (25 mM) | 3 | 300 |
| dNTP (2.5 mM) | 2.5 | 250 |
| DMSO | 0 | 0 |
| Primer 11a (10 µM) | 2 | 200 |
| Primer 11b (10 µM) | 2 | 200 |
| TaqGold (5 units/µl) | 0.3 | 30 |
| H₂O | 30.2 | 3020 |
| Sum: | 45 | 4500 |

5 µl genomic DNA (2ng/µl) was added to 45 µl PCR mix.

PCR cycling conditions for Eu11:
95°C 5 min, 50x(95°C 315s, 52°C 30s, 72°C 45s), 72°C 5 min, 4°C.

### Results

In this set of experiments 3 sequencing ("extension") primers for the RAAS system were used, and the signals resulting from the specific extension of each primer can be directly correlated to the number of nucleotides incorporated. Theoretical reference patterns are shown in Figure 10, which can be used to determine the genotype shown in figure 11. 10a, 10b and 10c are the theoretical outputs obtained for SNPs Eu8 (G/G), Eu7 (C/C and T/T) and Eu11 (A/G), with the theoretical multiplex output shown on figure 10d. This correlates to the actual results obtained shown in figure 11. The polymorphic positions are boxed, and the genotype of this individual is Eu8 G/G, Eu7 C/C and T/T and Eu11 A/G. The pyrogram exhibits some nucleotide background incorporation which can be reduced as discussed previously (e.g. add SSB after primer annealing).

### Example 4

SNP typing in Human Coagulation Factor V, Prothrombin and Plasminogen activator inhibitor.

### Introduction

Thrombosis is a complex (multifactorial) trait. The genes involved are typically susceptibility genes, where the differences are not point mutations but particular forms (alleles) of polymorphisms. The disorder results from the presence of an increased frequency of specific alleles in unfavorable combinations.

During the last ten to fifteen years, mutation or variation in several genes has been found to be associated with venous thrombosis. This includes genes such as factor V (FV), prothrombin (FII) and plasminogen activator inhibitor (PAI1).

Coagulation Factor V (FV) and Prothrombin (FII) are both essential components in the human coagulation cascade, which ultimately results in the stemming of blood loss. Prothrombin is proteolytically cleaved in the first step of this cascade converting into the clotting enzyme thrombin. Coagulation factor V serves as a cofactor for the coagulation factor X-catalyzed activation of prothrombin to thrombin. Point mutations in these genes may cause impairments in processes of thrombosis and hemostasis. One such is venous thrombosis, predominantly afflicting people of European origin. The mutations, Factor V Leiden (FV:G1691A) and the G20210-A prothrombin variant (FII:G20210A), are the two single most important genetic risk factors for developing venous thrombosis. This European predisposition has been explained to some extent by the characterization by these two variants. In addition to these two established risk factors for venous thrombosis, the role of other genetic variations is still under investigation (Martnelli et al., 1998; De Stefano et al., 1999; Rees et al., 1999; Hessner et al., 1999).

Several prospective studies have documented that the fibrinolytic capacity is an important determinant of the risk of thrombosis. Many studies have convincingly shown that survivors of myocardial infarction have impaired fibrinolytic activity because of increased concentrations of plasma plasminogen activator inhibitor-1 (PAI-1). A single guanosine insertion/deletion polymorphism in the promoter region of the PAI1 gene, commonly called 4G/5G, has been shown to be associated with plasma PAI-1 activity (Dawson et al, 1993; Eriksson et al., 1995).

### Primers

Three sets of PCR primers were designed. The fragment spanning over exon 10 and intron 10 of human coagulation factor V was 162 bp long, the prothrombin fragment spanning over exon 14 and intron 14 was 211 bp and the fragment in the promotor region of the PAI1 gene was 152 bp. One primer in each set was biotinylated in order to allow subsequent immobilization to magnetic beads/sepharose beads. In addition, three sequencing primers were designed to hybridize in close proximity to the factor V Leiden SNP, the G20210A prothrombin variant and the 4G/5G deletion of PAI1 see Figure 12.

### PCR primers:

| | |
|---|---|
| Prothrombin | |
| A001FPB | Biotin-5'-CCT GAA GAA GTG GAT ACA GAA GG-3' |
| | A008RP 5'-CAG TAG TAT TAC TGG CTC TTC CTG A-3' |
| Factor V | PSO90 5'-GGG CTA ATA GGA CTA CTT CTA ATC-3' |
| | PSO91B Biotin-5'-TCT CTT GAA GGA AAT GCC CCA TTA-3' |
| PAI1 | PSO112FPB Biotin-5'- CCC ACC CAG CAC ACC TC-3' |
| | PSO113RP 5'- GAC TCT TGG TCT TTC CCT CAT C-3' |

### Sequencing primers:

| | | |
|---|---|---|
| Prothrombin | A009SR | 5'-ACT GGG AGC ATT GAG-3' |
| Factor V | PSO83 | 5'-AGC AGA TCC CTG GAC-3' |
| PAI1 A114SR | | 5'- CAC GGC TGA CTC CCC-3' |

### PCR amplification

A 50 µl PCR reaction was set up using HotStarTaq Master Mix Kit from QiaGen according to the following protocol (Table 5).

**Table 5. Magnesium concentration 2.0 mM**

| **PCRmix** | **1x** | **100x** |
|---|---|---|
| 10xPCRbuffer (15 mM MgCl₂) | 5 | 500 |
| MgCl₂ (25 mM) | 4 | 400 |
| dNTP (2.5 mM) | 2.5 | 250 |
| A001FPB (10 mM) | 1 | 100 |
| A008RP (10 mM) | 1 | 100 |
| PSO90 (10 mM) | 1 | 100 |
| PSO91B (10 mM) | 1 | 100 |
| PSO112FPB (10 mM) | 1 | 100 |
| PSO113RP (10 mM) | 1 | 100 |
| HotStarTaq (5 units/ml) | 0.2 | 20 |
| H₂O | 29.3 | 2930 |
| Sum: | 45 | 4500 |

5 µl genomic DNA (2ng/µl) was added to 45 µl PCR mix. PCR cycling conditions:
95°C 5min, 50x(95°C 30s, 67°C 45s, 72°C 60s), 72°C 5min, 4°C.

### Sample preparation and primer extension

Were performed as described in Example 2.

### Results

The theoretical output obtained by typing each SNP or deletion individually are shown as figures 13a, 13b and 13c, representing PAI1 genotype for 4G/5G deletion (C/C), SNP G20210A prothrombin (C/T) and SNP G1691A Factor V Leiden (A/G), respectively. The theoretical multiplexing output for the multiplex assay of these 3 SNPs is shown as figure 13d, with the deletion or SNP position shown. Figures 13e to 13j represent the theoretical output expected for 6 genotypes upon which real data was then collected, see Figure 14. The pyrograms shown in figure 14 are 6 possible genotypes that can be present in the human population in these genes. 14a is the results from the genotype PAI1 C/C, prothrombin C/C and factor V G/G, 14b is the genotype PAI1 del/del, Prothrombin C/C and factor V A/A, 14c is the genotype PAI1 del/C, Prothrombin T/T and Factor V G/G, 14d is the genotype PAI C/C, prothrombin T/C and Factor V G/G, 14e is the genotype PAI1 C/del, Prothrombin C/C, Factor V G/G and 14f is the genotype PAI1 C/C, prothrombin C/C and Factor V A/G. Figure 14a corresponds to 13e, 14b to 13f, 14c to 13g, 14d to 13h, 14e to 13i and 14f to 13j.

### Example 5

### CYP2D6 SNP analysis

### Introduction

The CYP2D6 gene is a member of the cytochrome P450 gene superfamily, which in total consists of nine gene families. Four of these gene families are responsible for the metabolism and elimination of most foreign chemicals that enters the body via ingestion. The human CYP2D locus is mapped to chromosome 22q13.1 (Gough et.al, 1993). The CYP2D6 gene encodes for an enzyme, debrisoquine 4-hydroxylase, which is involved in the metabolism of more than 40 drugs, among them neuroleptics, antidepressants, anthiarrhytmics, b-blockers and opioids. The enzyme is characterised by extreme variability in activity (interindividual and interethnic). The CYP2D6 genotype and catalytic function are closely coupled, and genotyping could be an important tool for determining drug doses for individuals. More than 50 alleles have been identified, of which many encodes for a non-functional enzyme The alleles are defined by a number of variations; SNPs, insertion or deletions of single base pairs, deletion of the complete gene, and duplications of the gene. The sequences analysed in this example are as follows:
- G1846T:: GCCAACCACTCC G/T GT
- G1934A:: G/A GACGCCCCTTCG
- T1795del:: GCAG(T)GGGTGACCG
- G1749C:: G/C CTCCACCTTGCG

### Primers

Table 6. PCR primers and sequencing primers in the multiplex method. The primers are named F for a forward direction and R for a reversed direction. P represents a PCR primer, and S a sequencing primer and B means biotin labelled in the 5'end.

| Fragment | Primers | Primer sequence | Sequence to be identified |
|---|---|---|---|
| 61118 | A061RPB | B-CCTCGGTCTCTCGCTCCGC | |
| | A118FP | GAGCAGAGGCGCTTCTCCGT | |
| | A143FS | CCTTCGCCAACCAC | TCCG/TGT |
| | A182FS | CAAGAAGTCGCTGGAG | CAG (T) GGGTG |
| | A183FS | GCATCTCCCACCCCC | AG/AGACGCCCCTTTC |
| 2162 | A021RPB | B-ACTGTTTCCCAGATGGGCTC | |
| | A062FP | GACCCCGTTCTGTCTGGTGT | |
| | A145FS | TTCAATGATGAGAACC | TGC/TG |
| | A146FS | CCTGCTCATGATCCT | ACA/CTCCGG |
| | A147FS | TGAGCTGCTAACTGA | GCAC (A) GG |

Figure 15 shows the localisation of the primers in the CYP2D6 nucleic acid fragments for the multiplex method: fragments 2162 and 61118.

### PCR amplification

A nested PCR amplification was performed. For both the first and the nested 50 µl PCR reaction HotStarTaq Master Mix Kit from QiaGen was used and was set up according to the following protocol (Table 7).

**Table 7. Magnesium concentration 1.5 mM**

| **PCRmix** | **1x** | **100x** |
|---|---|---|
| 10xPCRbuffer (15 mM MgCl₂) | 5 | 500 |
| MgCl₂ (25 mM) | 0 | 0 |
| dNTP (2.5 mM) | 4 | 400 |
| Primer 1 (10 mM) | 1 | 100 |
| Primer 2 (10 mM) | 1 | 100 |
| HotStarTaq (5 units/µl) | 0.5 | 50 |
| H₂O | 37.5 | 3750 |
| Sum: | 49 | 4900 |

### PCR 1.

1 µl genomic DNA (10 ng/µl) was added to 49 µl PCR mix. PCR cycling conditions:
PCR method, primary PCR (fragment 4142)
95°C 15min, 25x(95°C 45s, 66°C 45s, 72°C 60s), 72°C 5min, 4°C
PCR method, secondary PCR
95°C 5min, 20x(95°C 45s, T_{A} 45s, 72°C 45s), 72°C 5min, 4°C
T_{A}, fragment 61118 was 61°C
2162 was 63°C

### Sample preparation

Took place as described in example 2. SSB was added to the primer/template mix after hybridisation. 0,55 µg SSB was added for fragment 2162 and 2,2 µg for fragment 61118. The amounts of sequencing primers were for fragment 2162: 15 pmoles of each, and for fragment 61118: 5 pmoles of primers 182 and 183, and 70 pmoles of primer 143.

### Primer extension

Was performed as described for example 2.

### Results

Two theoretical output for fragment 61118 in a multiplex analysis are shown as figures 16a and 16b. 16a showns the genotype G₁₉₃₄A (A/G), G₁₇₄₉C (C/G) , T₁₇₉₅del (no deletion) and G₁₈₄₆T (T/G) and figure 16b differs in that T₁₇₉₅del shows the deletion of the T residue.

Figure 17 shows the actual results from genotype established from the pyrogram is G₁₉₃₄A (G/G), G₁₇₄₉C (G/G), T₁₇₉₅del (no deletion T/T) and G₁₈₄₆T (G/G). This is a different genotype to those shown in figure 16. This demonstrates that it is possible to type multiple SNPs and deletions on one fragment of nucleic acid using multiple extension primers.

### EXAMPLE 6

### Serum samples

72 sera from HCV-positive Veterans were obtained from Stanford Veteran hospital. Five HCV-positive sera were obtained from Iran.

### Synthesis and purification of oligonucleotides

The oligonucleotides HCV-PCR-OUTF
(5'- CCCTGTGAGGAACTWCTGTCTTCACGC), HCV-PCR-OUTR
(5'-GCTCATGRTGCACGGTCTACGAGACCT), HCV-PCR-INF
(5'-TCTAGCCATGGCGTTAGTAYGAGTGT), BHCV-PCR-INR
(5'-Biotin-CACTCGCAAGCACCCTATCAGGCAGT), HCV-SEQF1
(5'-GGAACCGGTGAGTACACCGGAAT), HCV-SEQF2
(5'-GACYGGGTCCTTTCTTGGA), HCV-SEQF3
(5'-ATTTGGGCGTGCCCCCGC), were all synthesized and HPLC purified by MWG Biotech (High points, NC, USA).

### RNA extraction, cDNA synthesis and amplification

RNA was extracted from 50 µl of serum. cDNA was synthesized using AMV reverse transcriptase on HCV cDNA obtained from different patients using BHCV-PCR-INR and HCV-PCR-INF to generate a 270 base long product.

The biotinylated PCR products were immobilized onto streptavidin-coated super paramagnetic beads Dynabeads^{™} M280-Streptavidin (Dynal A.S., Oslo, Norway). Single-stranded DNA was obtained by removing the supernatant after incubation of the immobilized PCR product in 0.10 M NaOH for 3 min. Five pmol of sequencing primers HCV-SEQF1, HCV-SEQF2, and HCV-SEQF3 were hybridized to the immobilized strand.

### Primer extension reaction

The primed DNA template were placed in a microtiter plate containing 0.5 µg SSB (Amersham Pharmacia Biotech, USA), and Pyrosequencing^{™} substrates (www.pyrosequencing.com Pyrosequencing AB, Uppsala, Sweden) and enzymes were dispensed using fully automated microtiter plate-based PSQ^{™} Pyrosequencing^{™} instrument. The sequencing procedure was carried out by stepwise elongation of the primer-strand upon pre-specified addition of four different nucleotides. The template was hybridized with the three extension primers described above. The progress of sequencing was followed in real-time using Pyrosequencing^{™} SNP software, (Pyrosequencing^{™} AB, Uppsala, Sweden) and subtyping was performed manually.

### Results

### Principle of the typing method.

The principle of the typing method described above is outlined in Figure 1. In this model system, extension primers are hybridized to the target sample DNA, which is immobilized on magnetic beads.

The extension primers hybridise specifically to the conserved region adjacent to the variable region.

The signals resulting from the specific extension of each primer are directly correlated to the number of nucleotides incorporated. The 'fingerprint' produced can therefore be used to identify the genotype of the individual, against reference fingerprints, which can be theoretically deduced from the sequences of the variable regions. References fingerprints calculated theoretically from the sequence of the variable regions are shown on Figure 3. These can be used to type the results shown on Figure 4: Figure 4a is the fingerprint for HCV 1a, Figure 4b is the fingerprint for HCV 1b, Figure 4c is the fingerprint for HCV 2a, Figure 4d is the fingerprint for HCV 2b, Figure 4e is the fingerprint for HCV 3a, and Figure 4f is the fingerprint for HCV 3b. Therefore, using the method of the invention, it was possible to genotype HCV infection. Of the 77 sera analyzed by the method of the invention. 35% were infected with HCV 1a, 29% with HCV 1b, 21% with HCV 2a, 4% with HCV 2b, 10% with HCV 3a and 1% with HCV 3b.

## Claims

1. A method of typing one or more nucleic acid molecules, said method comprising:
simultaneously hybridizing two or more extension primers to said nucleic acid molecule(s), and performing primer extension reactions therefrom, each primer binding at a different predetermined site in said nucleic acid molecule(s) and wherein nucleotides are added to the reaction mix sequentially in a known or a predetermined order, and determining the pattern of nucleotide incorporation by sequencing-by-synthesis to obtain a test pattern for said nucleic acid molecule(s) which is optionally compared with one or more reference patterns to type the said nucleic acid molecule(s).

2. A method as claimed in claim 1 wherein the nucleic acid contains two or more variable sites.

3. A method as claimed in claim 1 for obtaining typing information about a plurality of variable sites within a target nucleic acid, said method comprising simultaneously hybridizing two or more extension primers to said target nucleic acid, and performing primer extension reactions therefrom, each primer binding at a different predetermined site in said target nucleic acid, the pattern of nucleotide incorporation, determined from said primer extension reactions by sequencing-by-synthesis, providing the typing information about said variable sites.

4. A method as claimed in any one of claims 1 to 3 wherein nucleotide incorporation is determined quantitatively.

5. A method as claimed in any one of claims 2 to 4 wherein if nucleotide incorporation takes place at one variable site, there is no nucleotide incorporation at the other variable site(s).

6. A method as claimed in any one of claims 2 to 5 wherein a first extension primer binds closer to its variable site than a second primer does to its variable site.

7. A method as claimed in claim 6 wherein the second primer is 10-20 nucleotides further away from its variable site than is said first primer.

8. A method as claimed in any one of claims 1 to 7 wherein single-stranded binding protein is added to the reaction mix after the primers are annealed to the nucleic acid template.

9. A method as claimed in any one of claims 1 to 8 wherein the primer extension reactions occur simultaneously.

10. A method as claimed in any one of claims 2 to 9 wherein 3 or more variable sites are typed.

11. A method as claimed in any one of claims 2 to 9 wherein 3 or more primer extension reactions are performed.

12. A method of diagnosis of pathological conditions **characterised by** the presence of specific nucleic acid molecule(s), said method comprising typing said nucleic acid molecule(s) by a method as defined in any one of claims 1 to 11, wherein the pattern of nucleotide incorporation allows diagnosis of said pathological conditions.

13. A method as claimed in any one of claims 1 to 12 wherein the pattern of nucleotide incorporation is determined simultaneously with the said primer extension, by cyclically or sequentially adding different nucleotides to the primer-extension reaction and detecting whether or not the added nucleotide is incorporated.

14. A method as claimed in any one of claims 1 to 13, wherein the pattern of nucleotide incorporation is determined by detecting pyrophosphate release.

15. A method as claimed in claim 14, wherein said pyrophosphate release is detected using the enzymes ATP sulphurylase and luciferase.

## Patentansprüche

1. Verfahren zur Typisierung eines oder mehrerer Nukleinsäuremoleküle, wobei man:
gleichzeitig zwei oder mehr Extensionsprimer mit dem Nukleinsäuremolekül (den Nukleinsäuremolekülen) hybridisiert, und davon Primer-Extensionsreaktionen durchführt, wobei jeder Primer an einer anderen, vorbestimmten Stelle in dem Nukleinsäuremolekül (den Nukleinsäuremolekülen) bindet und wobei Nukleotide dem Reaktionsgemisch sequentiell in einer bekannten oder vorbestimmten Reihenfolge zugegeben werden, und
das Muster des Nukleotideinbaus über Sequenzieren-durch-Synthese bestimmt, um ein Testmuster für das Nukleinsäuremolekül (die Nukleinsäuremoleküle) zu erhalten, das gegebenenfalls mit einem oder mehreren Referenzmustern verglichen wird, um das Nukleinsäuremolekül (die Nukleinsäuremoleküle) zu typisieren.

2. Verfahren nach Anspruch 1, wobei die Nukleinsäure zwei oder mehr variable Stellen enthält.

3. Verfahren nach Anspruch 1 zur Gewinnung von Typisierungsinformation über eine Pluralität von variablen Stellen innerhalb einer Ziel-Nukleinsäure, wobei man gleichzeitig zwei oder mehr Extensionsprimer mit der Ziel-Nukleinsäure hybridisiert, und davon Primer-Extensionsreaktionen durchführt, wobei jeder Primer an einer anderen, vorbestimmten Stelle in der Ziel-Nukleinsäure bindet, wobei das Muster des Nukleotideinbaus, bestimmt aus den Primer-Extensionsreaktionen über Sequenzieren-durch-Synthese, die Typisierungsinformation über die variablen Stellen bereit stellt.

4. Verfahren nach einem der Ansprüche 1 bis 3, wobei der Nukleotideinbau quantitativ bestimmt wird.

5. Verfahren nach einem der Ansprüche 2 bis 4, wobei im Falle von Nukleotideinbau an einer variablen Stelle kein Nukleotideinbau an der anderen variablen Stelle (den anderen variablen Stellen) erfolgt.

6. Verfahren nach einem der Ansprüche 2 bis 5, wobei ein erster Extensionsprimer näher an seiner variable Stelle bindet als ein zweiter Primer an seine variable Stelle bindet.

7. Verfahren nach Anspruch 6, wobei der zweite Primer 10-20 Nukleotide weiter von seiner variable Stelle entfernt ist als der erste Primer.

8. Verfahren nach einem der Ansprüche 1 bis 7, wobei ein Einzelstrang-Bindungsprotein dem Reaktionsgemisch zugegeben wird, nachdem ein Annealing der Primer mit dem Nukleinsäure-Template erfolgte.

9. Verfahren nach einem der Ansprüche 1 bis 8, wobei die Primer-Extensionsreaktionen gleichzeitig erfolgen.

10. Verfahren nach einem der Ansprüche 2 bis 9, wobei 3 oder mehr variable Stellen typisiert werden.

11. Verfahren nach einem der Ansprüche 2 bis 9, wobei 3 oder mehr Primer-Extensionsreaktionen durchgeführt werden.

12. Verfahren zur Diagnose pathologischer Zustände, die durch das Vorliegen von spezifischem Nukleinsäuremolekül (spezifischen Nukleinsäuremotekülen) **gekennzeichnet** sind, wobei man das Nukleinsäuremolekül (die Nukleinsäuremoleküle) über ein Verfahren gemäß der Definition in einem der Ansprüche 1 bis 11 typisiert, wobei das Muster des Nukleinsäureeinbaus eine Diagnose der pathologischen Zustände ermöglicht.

13. Verfahren nach einem der Ansprüche 1 bis 12, wobei das Muster des Nukleotideinbaus gleichzeitig mit der Primer-Extension bestimmt wird, indem zyklisch oder sequentiell unterschiedliche Nukleotide der Primer-Extensionsreaktion zugegeben werden und nachgewiesen wird, ob das zugegebene Nukleotid eingebaut wird oder nicht eingebaut wird.

14. Verfahren nach einem der Ansprüche 1 bis 13, wobei das Muster des Nukleotideinbaus durch Nachweis der Freisetzung von Pyrophosphat bestimmt wird.

15. Verfahren nach Anspruch 14, wobei die Freisetzung von Pyrophosphat unter Verwendung der Enzyme ATP-Sutfurylase und Luciferase nachgewiesen wird.

## Revendications

1. Procédé de typage d'une ou de plusieurs molécules d'acide nucléique, ledit procédé comprenant :
l'hybridation simultanée de deux ou plusieurs amorces d'extension à ladite ou aux dites molécules d'acide nucléique et la réalisation de réactions d'extension d'amorce à partir de celles-ci, chaque amorce se liant à un site prédéterminé différent au niveau de ladite ou desdites molécules d'acide nucléique, et dans lequel les nucléotides sont ajoutés au mélange réactionnel de manière séquentielle selon un ordre connu ou prédéterminé, et la détermination du profil d'incorporation de nucléotides par séquençage médié par synthèse en vue d'obtenir un modèle d'essai correspondant à ladite ou auxdites molécules d'acide nucléique qui est, le cas échéant, comparé à un ou à plusieurs modèles de référence pour typer ladite ou lesdites molécules d'acide nucléique.

2. Procédé selon la revendication 1, dans lequel l'acide nucléique contient deux ou plus de deux sites variables.

3. Procédé selon la revendication 1, pour l'obtention de données de typage concernant une pluralité de sites variables au sein d'un acide nucléique cible, ledit procédé comprenant l'hybridation simultanée de deux ou plus de deux amorces d'extension audit acide nucléique cible, et la réalisation de réactions d'extension d'amorce à partir de celui-ci, chaque amorce se liant à un site prédéterminé différent dans ledit acide nucléique cible, le profil d'incorporation de nucléotides, déterminé à partir desdites réactions d'extension d'amorce par séquençage médié par synthèse, fournissant les données de typage concernant lesdits sites variables.

4. Procédé selon l'une quelconque des revendications 1 à 3, dans lequel l'incorporation de nucléotides est déterminée de manière quantitative.

5. Procédé selon l'une quelconque des revendications 2 à 4, dans lequel, si l'incorporation de nucléotides a lieu au niveau d'un site variable, il n'y a pas d'incorporation de nucléotides au niveau d'un autre ou des autres sites variables.

6. Procédé selon l'une quelconque des revendications 2 à 5, dans lequel une première amorce d'extension se lie plus près de son site variable que ne le fait une seconde amorce vis-à-vis de son site variable.

7. Procédé selon la revendication 6, dans lequel la seconde amorce se trouve à raison de 10 à 20 nucléotides plus loin de son site variable que ne l'est ladite première amorce.

8. Procédé selon l'une quelconque des revendications 1 à 7, dans lequel la protéine de liaison simple brin est ajoutée au mélange réactionnel suite à l'hybridation des amorces à la matrice d'acide nucléique.

9. Procédé selon l'une quelconque des revendications 1 à 8, dans lequel les réactions d'extension d'amorce se produisent simultanément.

10. Procédé selon l'une quelconque des revendications 2 à 9, dans lequel trois ou plus de trois sites variables sont typés.

11. Procédé selon l'une quelconque des revendications 2 à 9, dans lequel trois ou plus de trois réactions d'extension d'amorce sont réalisées.

12. Procédé de diagnostic des états pathologiques **caractérisés par** la présence d'une ou des molécules d'acide nucléique spécifiques, ledit procédé comprenant le typage de ladite ou desdites molécules d'acide nucléique à l'aide d'un procédé tel que défini dans l'une quelconque des revendications 1 à 11, dans lequel le profil d'incorporation de nucléotides permet le diagnostic desdits états pathologiques.

13. Procédé selon l'une quelconque des revendications 1 à 12, dans lequel le profil d'incorporation de nucléotides est déterminé de manière simultanée à ladite extension d'amorce, par voie d'addition cyclique ou séquentielle des différents nucléotides au cours de la réaction d'extension d'amorce et la détection de l'incorporation ou non du nucléotide ajouté.

14. Procédé selon l'une quelconque des revendications 1 à 13, dans lequel le profil d'incorporation de nucléotides est déterminé en détectant la libération de pyrophosphate.

15. Procédé selon la revendication 14, dans lequel ladite libération de pyrophosphate est détectée en utilisant les enzymes ATP sulfurylase et luciférase.
